(19) Europäisches Patentamt
European Patent Office
Office européen des brevets

(11) **EP 2 905 288 B1**

(12) **EUROPÄISCHE PATENTSCHRIFT**

(45) Veröffentlichungstag und Bekanntmachung des Hinweises auf die Patenterteilung:
**26.12.2018 Patentblatt 2018/52**

(51) Int Cl.:
*C07K 7/06* (2006.01)         *A61K 38/08* (2006.01)
*A61K 38/10* (2006.01)         *C07K 7/08* (2006.01)

(21) Anmeldenummer: **15153281.9**

(22) Anmeldetag: **30.01.2015**

(54) **Synthetische artifizielle Peptide mit antimikrobieller Wirkung**

Synthetic artificial peptides having an anti-microbial effect

Peptide artificiel synthétique à effet antimicrobien

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **11.02.2014 DE 102014101663**

(43) Veröffentlichungstag der Anmeldung:
**12.08.2015 Patentblatt 2015/33**

(73) Patentinhaber: **Fraunhofer-Gesellschaft zur Förderung der angewandten Forschung e.V.
80686 München (DE)**

(72) Erfinder:
• **Rapsch, Karsten
13347 Berlin (DE)**
• **v. Nickisch-Rosenegk, Markus
14979 Großbeeren (DE)**

(74) Vertreter: **Andresen, Heiko
Hansepatent Patentanwälte
Andresen Scholz PartG mbB
Poststraße 33
20354 Hamburg (DE)**

(56) Entgegenhaltungen:
**EP-A1- 2 168 590     DE-T5-112011 100 556**

• **U. PAG ET AL: "In vitro activity and mode of action of diastereomeric antimicrobial peptides against bacterial clinical isolates", JOURNAL OF ANTIMICROBIAL CHEMOTHERAPY, Bd. 53, Nr. 2, 1. Februar 2004 (2004-02-01), Seiten 230-239, XP055187495, ISSN: 0305-7453, DOI: 10.1093/jac/dkh083**
• **MAEDA Y ET AL: "Helix-stabilizing effects of the pentapeptide KIFMK and its related peptides on the sodium channel inactivation gate peptides", JOURNAL OF PEPTIDE RESEARCH, Bd. 58, Nr. 5, November 2001 (2001-11), Seiten 413-423, XP002739311, ISSN: 1397-002X**
• **KARSTEN RAPSCH ET AL: "Rational Design of Artificial [beta]-Strand-Forming Antimicrobial Peptides with Biocompatible Properties", MOLECULAR PHARMACEUTICS, Bd. 11, Nr. 10, 6. Oktober 2014 (2014-10-06), Seiten 3492-3502, XP055187376, ISSN: 1543-8384, DOI: 10.1021/mp500271c**

SEQUENCE LISTING

<110> Fraunhofer Gesellschaft zur Foerderung der angewandten Forschung e. V.

<120> Synthetic artificial peptides with antimicrobial effect

<130> Synthizide

<160> 79

<170> PatentIn version 3.5

<210> 1
<211> 11
<212> PRT
<213> Artificial

<220>
<223> Synthetic peptide M1

<400> 1

```
              Lys Arg Lys Ile Leu Ile Leu Ile Lys Arg Lys
              1               5                   10
```

<210> 2
<211> 13
<212> PRT
<213> Artificial

<220>
<223> Synthetic peptide N1

<400> 2

```
          Lys Arg Lys Arg Lys Ile Leu Ile Leu Ile Lys Arg Lys
          1               5                   10
```

<210> 3
<211> 12
<212> PRT
<213> Artificial

<220>
<223> Synthetic Peptide N2

<400> 3

```
            Arg Lys Arg Lys Ile Leu Ile Leu Ile Lys Arg Lys
            1               5                   10
```

<210> 4
<211> 10
<212> PRT
<213> Artificial

<220>
<223> Synthetic Peptide N3

<400> 4

```
                    Arg Lys Ile Leu Ile Leu Ile Lys Arg Lys
                    1               5                   10
```

<210> 5
<211> 9
<212> PRT
<213> Artificial

<220>
<223> Synthetic peptide N4

<400> 5

```
                    Lys Ile Leu Ile Leu Ile Lys Arg Lys
                    1               5
```

<210> 6
<211> 13
<212> PRT
<213> Artificial

<220>
<223> Synthetic peptide C1

<400> 6

```
              Lys Arg Lys Ile Leu Ile Leu Ile Lys Arg Lys Arg Lys
              1               5                   10
```

<210> 7
<211> 12
<212> PRT
<213> Artificial

<220>
<223> Synthetic peptide C2

<400> 7

```
              Lys Arg Lys Ile Leu Ile Leu Ile Lys Arg Lys Arg
              1               5                   10
```

<210> 8
<211> 10
<212> PRT
<213> Artificial

<220>

<223> Synthetic peptide C3

<400> 8

                    Lys Arg Lys Ile Leu Ile Leu Ile Lys Arg

                    1                  5                  10

<210> 9
<211> 9
<212> PRT
<213> Artificial

<220>
<223> Synthetic peptide C4

<400> 9

                    Lys Arg Lys Ile Leu Ile Leu Ile Lys
                    1                  5

<210> 10
<211> 13
<212> PRT
<213> Artificial

<220>
<223> Synthetic peptide H1

<400> 10

                Lys Arg Lys Ile Leu Ile Leu Ile Leu Ile Lys Arg Lys
                1                  5                     10

<210> 11
<211> 10
<212> PRT
<213> Artificial

<220>
<223> Synthetic peptide H2

<400> 11

                    Lys Arg Lys Leu Ile Leu Ile Lys Arg Lys
                    1                  5                  10

<210> 12
<211> 9
<212> PRT
<213> Artificial

<220>
<223> Synthetic peptide H3

<400> 12

```
                    Lys Arg Lys Ile Leu Ile Lys Arg Lys
                    1               5
```

<210> 13
<211> 14
<212> PRT
<213> Artificial

<220>
<223> Synthetic peptide A1

<400> 13

```
            Arg Lys Arg Lys Leu Ile Leu Ile Leu Ile Lys Arg Lys Arg
            1               5                   10
```

<210> 14
<211> 14
<212> PRT
<213> Artificial

<220>
<223> Synthetic peptide A2

<400> 14

```
            Lys Arg Lys Leu Ile Leu Lys Arg Ile Leu Ile Lys Arg Lys
            1               5                   10
```

<210> 15
<211> 11
<212> PRT
<213> Artificial

<220>
<223> Synthetic peptide S1

<400> 15

```
                Arg Arg Arg Ile Ile Ile Ile Ile Arg Arg Arg
                1               5                   10
```

<210> 16
<211> 11
<212> PRT
<213> Artificial

<220>
<223> Synthetic peptide S2

<400> 16

```
                Lys Lys Lys Ile Ile Ile Ile Ile Lys Lys Lys
                1               5                   10
```

<210> 17
<211> 11
<212> PRT

<213> Artificial

<220>
<223> Synthetic peptide S3

<400> 17

```
                    Arg Arg Arg Leu Leu Leu Leu Leu Arg Arg Arg

                     1                   5                   10
```

<210> 18
<211> 11
<212> PRT
<213> Artificial

<220>
<223> Synthetic peptide S4

<400> 18

```
                    Lys Lys Lys Leu Leu Leu Leu Leu Lys Lys Lys
                     1                   5                   10
```

<210> 19
<211> 11
<212> PRT
<213> Artificial

<220>
<223> Synthetic peptide S5

<400> 19

```
                    Arg Arg Arg Val Val Val Val Val Arg Arg Arg
                     1                   5                   10
```

<210> 20
<211> 11
<212> PRT
<213> Artificial

<220>
<223> Synthetic peptide S6

<400> 20

```
                    Lys Lys Lys Val Val Val Val Val Lys Lys Lys
                     1                   5                   10
```

<210> 21
<211> 11
<212> PRT
<213> Artificial

<220>

<223> Synthetic peptide S7

<400> 21

```
                    Arg Arg Arg Phe Phe Phe Phe Phe Arg Arg Arg
                    1               5                   10
```

<210> 22
<211> 11
<212> PRT
<213> Artificial

<220>
<223> Synthetic peptide S8

<400> 22

```
                    Lys Lys Lys Phe Phe Phe Phe Phe Lys Lys Lys
                    1               5                   10
```

<210> 23
<211> 15
<212> PRT
<213> Artificial

<220>
<223> Synthetic peptide D4

<400> 23

```
            Lys Lys Lys Lys Lys Ile Ile Ile Ile Ile Lys Lys Lys Lys Asp
            1               5                   10                  15
```

<210> 24
<211> 15
<212> PRT
<213> Artificial

<220>
<223> Synthetic peptide D5

<400> 24

```
            Lys Lys Lys Lys Lys Ile Ile Ile Ile Ile Lys Lys Lys Lys Lys
            1               5                   10                  15
```

<210> 25
<211> 15
<212> PRT
<213> Artificial

<220>
<223> Synthetic peptide D6

<400> 25

```
            Lys Lys Lys Lys Lys Ile Ile Ile Ile Ile Asp Lys Lys Lys Lys
            1               5                   10                  15
```

<210> 26
<211> 15
<212> PRT
<213> Artificial

<220>
<223> Synthetic peptide D7

<400> 26

```
Lys Lys Lys Lys Lys Ile Ile Ile Ile Ile Asp Asp Asp Lys Lys
1               5                   10                  15
```

<210> 27
<211> 12
<212> PRT
<213> Artificial

<220>
<223> Synthetic peptide O1 and O1'

<400> 27

```
Lys Lys Lys Ile Ile Ile Ile Ile Ile Lys Lys Lys
1               5                   10
```

<210> 28
<211> 11
<212> PRT
<213> Artificial

<220>
<223> Synthetic peptide Pe01

<400> 28

```
Lys Arg Lys Ile Leu Ile Leu Ile Lys Arg Lys
1               5                   10
```

<210> 29
<211> 11
<212> PRT
<213> Artificial

<220>
<223> Synthetic peptide Pe02

<400> 29

```
Lys Arg Lys Phe Ile Phe Ile Phe Lys Arg Lys
1               5                   10
```

<210> 30
<211> 15
<212> PRT

<213> Artificial

<220>
<223> Synthetic peptide Pe10

<400> 30

```
        Lys Lys Lys Trp Lys Ile Val Val Ile Arg Lys Lys Lys Arg Lys
        1               5               10              15
```

<210> 31
<211> 7
<212> PRT
<213> Artificial

<220>
<223> Synthetic peptide Z1

<400> 31

```
                        Arg Lys Leu Ile Leu Lys Arg
                        1               5
```

<210> 32
<211> 14
<212> PRT
<213> Artificial

<220>
<223> Synthetic peptide Z2

<400> 32

```
        Lys Lys Lys Lys Lys Ile Ile Ile Lys Lys Lys Lys Lys Lys
        1               5               10
```

<210> 33
<211> 15
<212> PRT
<213> Artificial

<220>
<223> Synthetic peptide Z3

<400> 33

```
        Lys Lys Lys Lys Lys Ile Ile Ile Ile Lys Lys Lys Lys Lys Lys
        1               5               10              15
```

<210> 34
<211> 8
<212> PRT
<213> Artificial

<220>
<223> Synthetic peptide Z4

<400> 34

```
                        Lys Lys Ile Ile Ile Ile Lys Lys
                        1               5
```

<210> 35
<211> 10
<212> PRT
<213> Artificial

<220>
<223> Synthetic peptide Z5

<400> 35

```
                  Lys Lys Lys Ile Ile Ile Ile Lys Lys Lys


                  1               5                   10
```

<210> 36
<211> 12
<212> PRT
<213> Artificial

<220>
<223> Synthetic peptide Z6

<400> 36

```
            Lys Lys Lys Lys Ile Ile Ile Ile Lys Lys Lys Lys
            1               5                   10
```

<210> 37
<211> 13
<212> PRT
<213> Artificial

<220>
<223> Synthetic peptide Z7

<400> 37

```
            Lys Lys Lys Ile Ile Ile Ile Ile Ile Ile Lys Lys Lys
            1               5                   10
```

<210> 38
<211> 14
<212> PRT
<213> Artificial

<220>
<223> Synthetic peptide Z8

<400> 38

```
            Arg Arg Arg Arg Arg Ile Ile Ile Arg Arg Arg Arg Arg Arg
            1               5                   10
```

<210> 39
<211> 15
<212> PRT
<213> Artificial

<220>
<223> Synthetic peptide Z9

<400> 39

```
        Arg Arg Arg Arg Arg Ile Ile Ile Ile Arg Arg Arg Arg Arg Arg
        1                   5                   10                  15
```

<210> 40
<211> 8
<212> PRT
<213> Artificial

<220>
<223> Synthertic peptide Z10

<400> 40

```
                    Arg Arg Ile Ile Ile Ile Arg Arg
                    1               5
```

<210> 41
<211> 10
<212> PRT
<213> Artificial

<220>
<223> Synthetic peptide Z11

<400> 41

```
                Arg Arg Arg Ile Ile Ile Ile Arg Arg Arg
                1               5                   10
```

<210> 42
<211> 12
<212> PRT
<213> Artificial

<220>
<223> Synthetic peptide Z12

<400> 42

```
            Arg Arg Arg Arg Ile Ile Ile Ile Arg Arg Arg Arg
            1               5                   10
```

<210> 43
<211> 12
<212> PRT
<213> Artificial

<220>

<223> Synthetic peptide Z13

<400> 43

```
                    Arg Arg Arg Ile Ile Ile Ile Ile Ile Arg Arg Arg
                    1               5                       10
```

<210> 44
<211> 12
<212> PRT
<213> Artificial

<220>
<223> Synthetic peptide Z14

<400> 44

```
                    Arg Ile Arg Ile Ile Ile Ile Ile Ile Arg Ile Arg

                        1                   5                       10
```

<210> 45
<211> 9
<212> PRT
<213> Artificial

<220>
<223> Synthetic peptide Z15

<400> 45

```
                        Lys Lys Lys Ile Ile Ile Lys Lys Lys
                        1               5
```

<210> 46
<211> 9
<212> PRT
<213> Artificial

<220>
<223> Synthetic peptide Z16

<400> 46

```
                        Arg Arg Arg Ile Ile Ile Arg Arg Arg
                        1               5
```

<210> 47
<211> 15
<212> PRT
<213> Artificial

<220>
<223> Synthetic peptide Z17

<400> 47

```
                Lys Lys Lys Lys Lys Ile Val Val Ile Lys Lys Lys Lys Lys Lys
                1               5               10                  15
```

<210> 48
<211> 11
<212> PRT
<213> Artificial

<220>
<223> Synthetic peptide Z18

<400> 48

```
                    Lys Arg Lys Phe Ile Phe Ile Phe Lys Arg Lys
                    1               5                   10
```

<210> 49
<211> 13
<212> PRT
<213> Artificial

<220>
<223> Synthetic peptide Z19

<400> 49

```
                    Arg Arg Arg Ile Ile Ile Ile Ile Ile Ile Arg Arg Arg
                    1               5                   10
```

<210> 50
<211> 26
<212> PRT
<213> Artificial

<220>
<223> Melittin

<400> 50

```
            Gly Ile Gly Ala Val Leu Lys Val Leu Thr Thr Gly Leu Pro Ala Leu
            1               5               10                  15


            Ile Ser Trp Ile Lys Arg Lys Arg Gln Gln
                        20                  25
```

<210> 51
<211> 27
<212> PRT
<213> Artificial

<220>
<223> BMAP-27

<400> 51

42

```
Gly Arg Phe Lys Arg Phe Arg Lys Lys Phe Lys Lys Leu Phe Lys Lys
1               5               10              15

Leu Ser Pro Val Ile Pro Leu Leu His Leu Gly
            20              25
```

<210> 52
<211> 32
<212> PRT
<213> Artificial

<220>
<223> Protamine

<400> 52

```
Met Pro Arg Arg Arg Arg Ser Ser Ser Arg Pro Val Arg Arg Arg Arg
1               5               10              15

Arg Pro Arg Val Ser Arg Arg Arg Arg Arg Arg Gly Gly Arg Arg Arg
            20              25              30
```

<210> 53
<211> 13
<212> PRT
<213> Artificial

<220>
<223> Indolicidine

<400> 53

```
Ile Leu Pro Trp Lys Trp Pro Trp Trp Pro Trp Arg Arg
1               5               10
```

<210> 54
<211> 15
<212> PRT
<213> Artificial

<220>
<223> KL-Helix

<400> 54

```
Leu Lys Leu Leu Lys Lys Leu Leu Lys Lys Leu Leu Lys Leu Leu
1               5               10              15
```

<210> 55
<211> 11
<212> PRT
<213> Artificial

<220>
<223> Synthetic peptide M2

<400> 55

```
                        Lys Arg Lys Ile Leu Ile Leu Ile Leu Ile Leu
                        1               5                   10
```

<210> 56
<211> 11
<212> PRT
<213> Artificial

<220>
<223> Synthetic peptide M3

<400> 56

```
                        Ile Leu Ile Leu Ile Leu Ile Leu Lys Arg Lys
                        1               5                   10
```

<210> 57
<211> 11
<212> PRT
<213> Artificial

<220>
<223> Synthetic peptide M4

<400> 57

```
                        Lys Arg Lys Ile Leu Ile Leu Ile Gly Ser Gly
                        1               5                   10
```

<210> 58
<211> 11
<212> PRT
<213> Artificial

<220>
<223> Synthetic peptide M5

<400> 58

```
                        Gly Ser Gly Ile Leu Ile Leu Ile Lys Arg Lys
                        1               5                   10
```

<210> 59
<211> 11
<212> PRT
<213> Artificial

<220>
<223> Synthetic peptide M6

<400> 59

```
                        Lys Arg Lys Ser Gly Ser Gly Ser Lys Arg Lys
                        1               5                   10
```

<210> 60
<211> 8
<212> PRT
<213> Artificial

<220>
<223> Synthetic peptide N5

<400> 60

```
Ile Leu Ile Leu Ile Lys Arg Lys
1               5
```

<210> 61
<211> 7
<212> PRT
<213> Artificial

<220>
<223> Synthetic peptide N6

<400> 61

```
Leu Ile Leu Ile Lys Arg Lys
1               5
```

<210> 62
<211> 6
<212> PRT
<213> Artificial

<220>
<223> Synthetic peptide N7

<400> 62

```
Ile Leu Ile Lys Arg Lys
1               5
```

<210> 63
<211> 5
<212> PRT
<213> Artificial

<220>
<223> Synthetic peptide N8

<400> 63

```
Leu Ile Lys Arg Lys
1               5
```

<210> 64
<211> 8
<212> PRT
<213> Artificial

<220>

<223> Synthetic peptide C5

<400> 64

Lys Arg Lys Ile Leu Ile Leu Ile
1               5

<210> 65
<211> 7
<212> PRT
<213> Artificial

<220>
<223> Synthetic peptide C6

<400> 65

Lys Arg Lys Ile Leu Ile Leu
1               5

<210> 66
<211> 6
<212> PRT
<213> Artificial

<220>
<223> Synthetic peptide C7

<400> 66

Lys Arg Lys Ile Leu Ile
1               5

<210> 67
<211> 5
<212> PRT
<213> Artificial

<220>
<223> Synthetic peptide C8

<400> 67

Lys Arg Lys Ile Leu
1               5

<210> 68
<211> 8
<212> PRT
<213> Artificial

<220>
<223> Synthetic peptide H4

<400> 68

Lys Arg Lys Leu Ile Lys Arg Lys
1               5

<210> 69
<211> 7
<212> PRT
<213> Artificial

<220>
<223> Synthetic peptide H5

<400> 69

```
Lys Arg Lys Ile Lys Arg Lys
1               5
```

<210> 70
<211> 6
<212> PRT
<213> Artificial

<220>
<223> Synthetic peptide H6

<400> 70

```
Lys Arg Lys Lys Arg Lys
1               5
```

<210> 71
<211> 7
<212> PRT
<213> Artificial

<220>
<223> Synthetic peptide H7

<400> 71

```
Lys Arg Lys Gly Lys Arg Lys
1               5
```

<210> 72
<211> 9
<212> PRT
<213> Artificial

<220>
<223> Synthetic peptide H8

<400> 72

```
Lys Arg Lys Gly Ser Gly Lys Arg Lys
1               5
```

<210> 73
<211> 14
<212> PRT
<213> Artificial

<220>

<223> Synthetic peptide A3

<400> 73

```
Arg Lys Leu Ile Leu Lys Arg Lys Arg Ile Leu Ile Lys Arg
1               5                   10
```

<210> 74
<211> 14
<212> PRT
<213> Artificial

<220>
<223> Synthetic peptid A4

<400> 74

```
Lys Leu Ile Leu Lys Arg Lys Arg Lys Arg Ile Leu Ile Lys
1               5                   10
```

<210> 75
<211> 14
<212> PRT
<213> Artificial

<220>
<223> Synthetic peptide A5

<400> 75

```
Leu Ile Leu Lys Arg Lys Arg Lys Arg Lys Arg Ile Leu Ile
1               5                   10
```

<210> 76
<211> 15
<212> PRT
<213> Artificial

<220>
<223> Synthetic peptide D1

<400> 76

```
Lys Lys Lys Lys Lys Ile Ile Ile Ile Ile Asp Asp Asp Asp Asp
1               5                   10                  15
```

<210> 77
<211> 15
<212> PRT
<213> Artificial

<220>
<223> Synthetic peptide D2

<400> 77

```
Lys Lys Lys Lys Lys Ile Ile Ile Ile Ile Lys Asp Asp Asp Asp
1               5                   10                  15
```

<210> 78
<211> 15
<212> PRT
<213> Artificial

<220>
<223> Synthetic peptide D3

<400> 78

```
Lys Lys Lys Lys Lys Ile Ile Ile Ile Ile Lys Lys Lys Asp Asp
1               5                   10                  15
```

<210> 79
<211> 15
<212> PRT
<213> Artificial

<220>
<223> Synthetic peptide D8

<400> 79

```
Lys Lys Lys Lys Lys Ile Ile Ile Ile Ile Asp Asp Asp Asp Lys
1               5                   10                  15
```

**Beschreibung**

Die vorliegende Erfindung betrifft Peptide mit antimikrobieller Wirkung, deren medizinische Verwendung sowie Konjugate und Zusammensetzungen umfassend solche Peptide.

Die Kontrolle und Behandlung bakterieller Infektionen ist ein Schwerpunktthema in der Gesundheitsversorgung. Über die vergangenen Jahrzehnte hat die intensive Nutzung von antibiotischen Wirkstoffen in der Humanmedizin, Veterinärmedizin und Landwirtschaft in zunehmendem Maße zur Ausbildung von antibiotikaresistenten Keimen geführt. Solche (multi)resistenten Keime stellen die moderne Gesundheitsversorgung heute sowohl medizinisch als auch ökonomisch vor große Probleme.

Auf der Suche nach Alternativen zu den herkömmlichen Antibiotika sind in den letzten Jahren antimikrobielle oder bakteriozide Peptide (AMPs) in den Mittelpunkt des wissenschaftlichen und medizinischen Interesses gerückt (Marr et al. 2006, Gordon et al. 2005). Es ist eine Reihe natürlich vorkommender Peptide mit antibakterieller Wirkung bekannt (Brogden et al. 2003, Diamond 2001, Lehrer and Ganz 2002, Tossi et al. 2000). Diese antimikrobiellen Peptide weisen zum Teil große Unterschiede in ihrer Sequenz auf, wobei die antimikrobiellen Eigenschaften im Wesentlichen mit zwei unterschiedlichen Wirkungsweisen in Zusammenhang gebracht werden. Eine bakteriozide Wirkung ist von Peptiden bekannt, welche aufgrund einer charakteristischen Konformationsannahme mit biologischen Membranen assoziieren und beispielsweise zu Membranpermeabilisierung führen (Shai 1999). Eine bakteriostatische Wirkung wird solchen Peptiden zugerechnet, welche durch Wechselwirkung mit zellinternen Komponenten, beispielsweise Enzymen, einen entsprechenden Funktionsverlust metabolischer Schlüsselkomponenten verursachen (Gennaro and Zanetti 2000, Brotz et al. 1998, Subbalakshmi and Sitaram 1998, Otvos et al. 2000, Hilpert et al. 2010, Brogden 2005).

Als nachteilig hat sich jedoch herausgestellt, dass natürliche antimikrobielle Peptide häufig starke zytotoxische Eigenschaften aufweisen (Conlon et al. 2003, Ahmad et al. 2009, Ginsburg and Koren 2008). Auch sind natürliche AMPs aufgrund ihrer häufig komplexen Struktur mit hohen Herstellungskosten und oft schwachen pharmakokinetischen Eigenschaften behaftet (Zaiou 2007, Giuliani et al. 2007, Oyston et al. 2009), so dass nur ein eingeschränkter kommerzieller und klinischer Nutzen besteht. Die therapeutische Verwendung natürlicher antimikrobieller Peptide könnte zudem ein grundsätzliches Gesundheitsrisiko darstellen, weil möglicherweise Resistenzen gegen die eng verwandten antimikrobiellen Peptide des innaten Immunsystems gefördert und auf diese Weise die natürliche Immunabwehr gegen bakterielle Infektionen gefährlich geschwächt werden würde (Bell and Gouyon 2003).

Ein alternatives Konzept stellt die Entwicklung nicht natürlich vorkommender Peptide mit antimikrobiellen Eigenschaften dar. Einen Forschungsschwerpunkt bildet hierbei das Design alpha-helikaler Peptide, da dieses Strukturmotiv oft in Zusammenhang mit den antimikrobiellen Eigenschaften von natürlichen AMPs gebracht wird (Dathe 1997). Ein Nachteil alpha-helikaler Peptide ist, dass diese neben ihrer effektiven antimikrobiellen Wirkung oft auch ausgeprägte zytotoxische Eigenschaften gegenüber eukaryotischen Zellen aufweisen (Dathe 1997, Wiradharma 2013).

Ein weitaus weniger verbreitetes Strukturmotiv antimikrobieller Peptide ist das amphiphatische beta-Faltblatt. Das Design von beta-Faltblattpeptiden mit antimikrobieller Wirkung beinhaltet üblicherweise eine alternierende Anordnung von hydrophoben und hydrophilen Aminosäuren (Ong et al. 2013, Rausch et al. 2005, Rausch et al. 2007, Jin et al. 2005, Pag et al. 2004).

Trotz intensiver Forschungsbemühungen weisen die bisher bekannten antimikrobiellen Peptide Nachteile auf.

Insbesondere stellen eine unzureichende antimikrobielle Wirkung, ein geringes Wirkungsspektrum, zytotoxische Nebeneffekte, eingeschränkte wirkungskinetische und/oder pharmakokinetische Eigenschaften sowie hohe Herstellungskosten limitierende Faktoren dar.

Eine zu lösende Aufgabe der vorliegenden Erfindung besteht daher darin, Peptide mit verbesserten Eigenschaften hinsichtlich einzelner oder mehrerer der oben genannten Nachteile bereitzustellen.

Weitere zu lösende Aufgaben der Erfindung bestehen darin, eine medizinische Verwendung der Peptide sowie Konjugate und antibakterielle Zusammensetzungen umfassend die Peptide anzugeben.

Dieses Ziel wird erfindungsgemäß durch ein isoliertes Peptid nach Anspruch 1 erreicht. Vorteilhafte Ausgestaltungen und Verwendungen des isolierten Peptids sind Gegenstand von weiteren Ansprüchen.

Der vorliegenden Erfindung liegt die Entwicklung eines artifiziellen peptidischen Strukturprinzips zugrunde, welches die Darstellung ungewöhnlicher und überraschend einfacher AMPs mit einer hohen, teilweise den bekannten und natürlichen Peptiden überlegenen antimikrobiellen Wirkung ermöglicht. Dabei ist es von besonderem Vorteil, dass die erfindungsgemäßen Peptide ein breites Wirkungsspektrum gegenüber gram(-) und gram(+) Bakterien aufweisen und gleichzeitig hochselektiv gegen Bakterienzellen wirken, das heißt, keine oder nur sehr geringe zytotoxische Eigenschaften haben.

Die Grundstruktur der erfindungsgemäßen Peptide ist durch einen N-terminalen Bereich A und einen C-terminalen Bereich C mit jeweils überwiegend basischen Aminosäuren gekennzeichnet, zwischen denen ein zentraler Bereich B mit überwiegend hydrophoben und/oder unpolaren Aminosäuren angeordnet ist.

In einer Ausführungsform der Erfindung ist das isolierte Peptid durch die allgemeine Formel

$$A_x B_y C_z$$

angegeben. Hierin sind A und C die flankierenden Bereiche mit überwiegend basischen Aminosäuren, wobei x und z für die Anzahl der Aminosäuren im Bereich A beziehungsweise C stehen. B ist der zentrale Bereich mit überwiegend hydrophoben und/oder unpolaren Aminosäuren. Entsprechend kennzeichnet y die Anzahl der Aminosäuren im Bereich B.

Die Ausgestaltungen der Bereiche A, B und C können beispielsweise hinsichtlich Kettenlänge und enthaltender Aminosäuren variieren, wobei x und z größer gleich 1 und y größer gleich 3 ist. Die Gesamtzahl der Aminosäuren x + y + z im Peptid ist kleiner gleich 50, kleiner gleich 35 oder kleiner gleich 25. Insbesondere gestattet das erfindungsgemäße Strukturmotiv aber auch die Ausführung sehr kurzer Peptide mit hoher antimikrobieller Wirkung.

Beispielsweise kann die Gesamtzahl x + y + z der Aminosäuren kleiner gleich 20, kleiner gleich 18 oder kleiner gleich 16 sein. Auf diese Weise wird eine maximierte antimikrobielle Wirkung mit einer einfachen und wirtschaftlichen Herstellung besonders vorteilhaft vereint.

A und C weisen jeweils unabhängig voneinander mindestens 65%, mindestens 75% oder mindestens 80% basische Aminosäuren auf. A und C können jeweils auch unabhängig voneinander mindestens 90% oder mindestens 95% basische Aminosäuren aufweisen oder ausschließlich aus basischen Aminosäuren bestehen.

B weist mindestens 80% hydrophobe und/oder unpolare Aminosäuren ausgewählt aus der Gruppe bestehend aus Val, Ile, Phe und beliebigen Kombinationen von Val, Leu, Ile und Phe auf. B kann auch mindestens 90% oder mindestens 95% hydrophobe und/oder unpolare Aminosäuren aufweisen oder ausschließlich aus hydrophoben und/oder unpolaren Aminosäuren bestehen.

Insbesondere liegt in B eine direkte Aufeinanderfolge von mindestens 3, 4 oder 5 hydrophoben und/oder unpolaren Aminosäuren vor. Eine direkte Aufeinanderfolge im Sinne der Erfindung liegt dann vor, wenn zwischen den hydrophoben und/oder unpolaren Aminosäuren keine weiteren Aminosäuren, zum Beispiel polare Aminosäuren, angeordnet sind.

Ein Peptid im Sinne der Erfindung liegt grundsätzlich dann vor, wenn die Aminosäuresequenz eines Peptids in verständiger Auslegung zumindest eine natürliche Lösung für x, y, und z liefert, bei der die Formel $A_x B_y C_z$ die oben genannten Merkmale erfüllt. Es sind x, y und z natürliche Zahlen. Die Formel ist dahingehend zu verstehen, dass neben den Bereichen A, B und C keine weiteren Bereiche vorhanden sind.

In gewissen Weiterbildungen enthalten A und/oder C unabhängig voneinander höchstens eine oder keine sauren Aminosäuren. Alternativ oder zusätzlich enthalten A und/oder C unabhängig voneinander höchstens eine oder keine hydrophobe Aminosäuren. B enthält in gewissen Weiterbildungen höchstens zwei, eine oder keine sauren Aminosäuren. Alternativ oder zusätzlich enthält B höchstens eine oder keine basische Aminosäuren. Wie allgemein in dem Fachgebiet üblich sind im Sinne der vorliegenden Erfindung Valin, Leucin, Isoleucin und Phenylalanin als hydrophobe beziehungsweise unpolare Aminosäuren zu verstehen. Entsprechend sind basische Aminosäuren Lysin, Arginin und Histidin. Demgegenüber sind die Aminosäuren Tyrosin, Threonin, Glutamin, Glycin, Serin, Cystein und Asparagin neutral beziehungsweise polar. Glutaminsäure und Asparaginsäure sind saure Aminosäuren. Nachfolgend werden die Aminosäuren auch im allgemein gebräuchlichen Einbeziehungsweise Dreibuchstabencode bezeichnet (Stryer 1999). Für den Fachmann ist leicht ersichtlich, dass die Erfindung nicht auf proteinogene Aminosäuren beschränkt ist. Insbesondere kann das isolierte Peptid auch entsprechende nichtproteinogene Aminosäuren umfassen. Beispielsweise können die entsprechenden D-Enantiomere, Diastereomere, beta- oder gamma-Aminosäurederivate der proteinogenen Aminosäuren enthalten sein. Nicht beschränkende Beispiele sind D-Val, D-Leu, D-Ile, D-Phe, D-Lys und D-Arg. Des Weiteren kann das isolierte Peptid auch synthetische Bausteine, beispielsweise mit entsprechenden basischen, zum Beispiel alpha-Amino-3-guanidino-Propionsäure, oder hydrophoben beziehungsweise unpolaren Eigenschaften, zum Beispiel Norleucin, sowie Carbamatverbindungen umfassen. Vorzugsweise sind diese über mindestens eine Amidbindung in die Peptidsequenz integriert. Es ist auch möglich, den C-Terminus und/oder N-Terminus des Peptids amidiert beziehungsweise acetyliert auszubilden. Auf diese Weise können zum Beispiel Peptide mit verbesserten pharmakokinetischen Eigenschaften und/oder geringerer Gefahr von Resistenzentwicklungen bereitgestellt werden, da sie den natürlichen Erkennungs- und Abbaumechanismen weitgehend entzogen sind.

Die Erfinder konnten feststellen, dass solche Peptide besonders gute antimikrobielle Eigenschaften und gleichzeitig geringe zytotoxische Wirkung haben.

In einer bevorzugten Ausführungsform sind die basischen Aminosäuren unabhängig voneinander ausgewählt aus Lysin und Arginin und Kombinationen davon. Besonders bevorzugt ist Lysin. Auf diese Weise kann zum Beispiel die Selektivität gegenüber mikrobiellen Zellen erhöht und die Zytotoxizität verringert werden.

Die Anzahl x der Aminosäuren im aminoterminalen Bereich A ist vorzugsweise x größer gleich 2 oder x größer gleich 3. Alternativ oder zusätzlich ist die Anzahl z der Aminosäuren im carboxyterminalen Bereich C größer gleich 2 oder größer gleich 3. In einer bevorzugten Weiterbildung sind zudem x und z jeweils unabhängig voneinander kleiner gleich 12, kleiner gleich 9 oder kleiner gleich 7.

Typischerweise weist der Bereich B eine Anzahl y von größer gleich 4 oder größer gleich 5 Aminosäuren auf. In bestimmten Ausführungsformen ist vorgesehen, dass die Gesamtzahl y der Aminosäuren im zentralen Bereich B kleiner gleich 20, kleiner gleich 15 oder kleiner gleich 10 ist. In diesen Ausgestaltungen wird beispielsweise eine besonders gute antimikrobielle Wirkung gegen gram(-) negative Bakterien erreicht.

In bestimmten Ausführungsformen ist die N-terminale Aminosäure in A eine basische Aminosäure. Alternativ oder zusätzlich ist die C-terminale Aminosäure in C eine basische Aminosäure. Mit solchen Peptiden konnten die Erfinder besonders gute antimikrobielle Eigenschaften erreichen.

Vorzugsweise ist das Peptid in einem wässrigen Milieu zumindest teilweise als beta-Strang ausgebildet. Die Erfinder konnten feststellen, dass ein unmittelbarer Zusammenhang zwischen dem beta-Strang Gehalt der erfindungsgemäßen Peptide und ihrer antimikrobiellen Wirksamkeit besteht. Dies ist insofern überraschend, da bislang bekannte beta-Faltblatt Motive in antimikrobiellen Peptiden üblicherweise eine Wechselfolge von hydrophoben Aminosäuren und hydrophilen, ungeladenen oder geladenen Aminosäuren erfordern (siehe zum Beispiel Rausch et al. 2007; Ong et al. 2013). Demgegenüber gelingt den Erfindern über das vorliegende Strukturmotiv die Ausbildung ungewöhnlicher beta-Strang Peptide. Auf diese Weise können kürzere und/oder hinsichtlich der Aminosäurezusammensetzung einfachere Peptide mit antimikrobiell wirksamer beta-Struktur bereitgestellt werden.

Geeignete Methoden zur Bestimmung des beta-Strang Gehalts der erfindungsgemäßen Peptide sind in Beispiel 8 erläutert. Alternative analytische Methoden wie zum Beispiel Circulardichroismus oder Infrarotspektroskopie sind dem Fachmann hinlänglich bekannt (Lottspeich and Engels 2006; Richter 2003) .

Insbesondere können mit der erfindungsgemäßen Struktur Peptide mit einem hohen beta-Strang Gehalt realisiert werden. Die Erfinder konnten nachweisen, dass ein hoher beta-Strang Gehalt in den erfindungsgemäßen Peptiden mit einer hohen antimikrobiellen Wirkung und geringen Zytotoxizität korreliert. In bevorzugten Ausführungsformen sind daher mindestens 50%, 65%, 75% oder 85% der Aminosäuren des Peptids in einem einzigen beta-Strang umfasst. Auf diese Weise werden Peptide bereitgestellt, welche bereits in niedrigen Konzentrationen und/oder innerhalb kurzer Zeit Bakterienmembranen zerstören.

In einer weiterhin bevorzugten Ausführungsform liegt das Peptid als gestreckter beta-Strang vor. Ein gestreckter beta-Strang im Sinne der Erfindung liegt insbesondere dann vor, wenn das Peptid einen durchgehenden beta-Strang ausbildet, welcher nicht durch eine beta-Schleife, alpha-Helix und/oder unstrukturierte Region unterbrochen ist. Da im Stand der Technik die porenbildende Eigenschaft von AMPs bisher lediglich mit einer beta-Faltblattstruktur in Zusammenhang gebracht wird, bei der wenigstens zwei beta-Stränge eines Peptids über eine beta-Schleife räumlich wechselwirken (Rausch et al. 2005; Rausch et al. 2007), bietet es unerwartete Vorteile, dass solche Strukturen in den erfindungsgemäßen Peptiden nicht erforderlich sind. Beispielsweise können deutlich kürzere und somit wirtschaftlichere AMPs bereitgestellt werden, welche teilweise sogar höhere antimikrobielle Aktivität als die herkömmlichen beta-Faltblatt strukturierten AMPs aufweisen.

In bestimmten Ausführungsformen weist das erfindungsgemäße Peptid darüber hinaus in einem wässrigen Milieu keine alpha-Helix auf. Durch die Vermeidung von alpha-helikalen Motiven im Peptid können erfindungsgemäß zytotoxische Nebenwirkungen der AMPs wirksam vermieden werden.

Gemäß einer weiteren Ausführungsform ist das Peptid am C-Terminus als Carbonsäureamid ausgebildet. Die Erfinder konnten überraschend feststellen, dass mit Hilfe einer Amidierung des Carboxyterminus gegenüber der entsprechenden freien Carbonsäure der erfindungsgemäßen Peptide eine weitere Verbesserung der antimikrobiellen Eigenschaften erreicht wird.

In einer weiteren Ausführungsform der Erfindung weist das Peptid eine Anzahl x beziehungsweise eine Anzahl z von Aminosäuren im aminoterminalen Bereich A beziehungsweise carboxyterminalen Bereich C von $3 \leq x \leq 7$ beziehungsweise $3 \leq z \leq 7$ auf. Gleichzeitig weist das Peptid eine Anzahl y von Aminosäuren im dazwischenliegenden Bereich B von $5 \leq y \leq 10$ auf. Demgemäß können, in beliebiger Kombination, unabhängig voneinander x und z jeweils 3, 4, 5, 6 oder 7 und y 5, 6, 7, 8, 9 oder 10 sein. In einer bevorzugten Weiterbildung dieser Ausführungsform sind die Aminosäuren von A und C unabhängig voneinander ausgewählt aus der Gruppe bestehend aus Lysin und Arginin sowie Kombinationen dieser Aminosäuren. Des Weiteren sind die Aminosäuren von B ausgewählt aus der Gruppe bestehend aus Valin, Isoleucin und Phenylalanin sowie Kombinationen von Valin, Leucin, Isoleucin und Phenylalanin. In diesen Ausgestaltungen vereinen die erfindungsgemäßen Peptide besonders hohe antimikrobielle Wirksamkeit und ein breites Wirkungsspektrum mit minimaler Zytotoxizität. Gleichzeitig ist aufgrund der Kürze der Sequenz eine besonders wirtschaftliche Herstellbarkeit gewährleistet.

In einer Ausführungsform weist das Peptid eine minimale Hemmkonzentration (MHK) gegenüber gram(-) Bakterien von kleiner gleich 32 $\mu$M, kleiner gleich 16 $\mu$M, kleiner gleich 8 $\mu$M, kleiner gleich 4 $\mu$M, kleiner gleich 2 $\mu$M, kleiner gleich 1 $\mu$M oder kleiner gleich 0,5 $\mu$M auf. Alternativ oder zusätzlich kann das Peptid eine minimale Hemmkonzentration (MHK) gegenüber gram(+) Bakterien von kleiner gleich 32 $\mu$M, kleiner gleich 16 $\mu$M, kleiner gleich 8 $\mu$M, kleiner gleich 4 $\mu$M, kleiner gleich 2 $\mu$M, kleiner gleich 1 $\mu$M oder kleiner gleich 0,5 $\mu$M aufweisen. Eine geeignete Methode zur Bestimmung der MHK ist weiter unten in Beispiel 2 beschrieben.

In einer weiteren Ausführung der Erfindung besitzt das Peptid die Fähigkeit, die Membran von gram(+) Bakterien ab einer Peptidkonzentration von 50 $\mu$M, 25 $\mu$M oder 5 $\mu$M zu permeabilisieren. Alternativ oder zusätzlich besitzt das Peptid die Fähigkeit, die Membran von gram(-) Bakterien ab einer Peptidkonzentration von 50 $\mu$M, 25 $\mu$M oder 5 $\mu$M

zu permeabilisieren. Eine geeignete Methode zur Bestimmung der membranpermeabilisierenden Peptidkonzentration ist in Beispiel 4 beschrieben.

Die $LD_{50}$ gegenüber eukaryotischen Zellen des Peptids liegt vorzugsweise bei einer Konzentration größer gleich 50 μM, größer gleich 60 μM oder größer gleich 64 μM. Eine geeignete Methode zur Bestimmung des $LD_{50}$-Wertes ist in Beispiel 6 erläutert. Solche Peptide sind aufgrund geringer zytotoxischer Nebenwirkungen besonders für die therapeutische Antibiose geeignet.

Ein weiterer Aspekt der Erfindung betrifft ein Konjugat umfassend das vorangehend beschriebene Peptid. Ein Konjugat im Sinne der Erfindung ist ein Material umfassend das Peptid und zumindest eine weitere Komponente. Die weitere Komponente weist gegenüber dem Peptid wenigstens eine abweichende Eigenschaft auf, welche typischerweise eine physikalische, strukturelle und/oder (bio-)chemische Eigenschaft ist. Dadurch erhält das erfindungsgemäße Konjugat zumindest teilweise andere Eigenschaften als seine einzelnen Komponenten jeweils für sich alleine genommen. Insbesondere liegt das Peptid an die weitere Komponente kovalent und/oder adsorptiv gekoppelt vor. Die weitere Komponente kann zum Beispiel ein (Signal-)Peptid, ein Protein, eine Nukleinsäure, ein Lipid oder ein Saccharid umfassen. Die weitere Komponente kann auch einen Partikel wie beispielsweise einen organischen oder anorganischen Nano- oder Mikropartikel umfassen. Auf diese Weise können mit dem erfindungsgemäßen Konjugat verschiedene vorteilhafte Effekte erreicht werden, zum Beispiel eine verbesserte Bioverfügbarkeit und/oder Biokompatibilität, eine zielgerichtete beziehungsweise selektive Anreicherung oder Freisetzung des Peptids am gewünschten Wirkungsort.

Die weitere Komponente kann auch natürliche und/oder synthetische Polymere umfassen. Geeignete Polymere sind, ohne Beschränkung, Polyethylenglykole (PEG), Acrylate, Epoxide, Urethane, Zellulosederivate, Polyester, Polystryrol- und Polyvinylpolymere, Silikone, Parylene und Kombinationen davon. Des Weiteren sind als weitere Komponente auch funktionalisierte Oberflächen wie zum Beispiel glas-, kunststoff- oder metallumfassende Oberflächen vorgesehen.

Ein weiterer Aspekt der Erfindung betrifft die medizinische Verwendung des vorangehend beschriebenen Peptids als Medikament. Insbesondere ist die medizinische Verwendung des Peptids zur Behandlung von bakteriellen Infektionen vorgesehen. Eine solche bakterielle Infektion kann zum Beispiel einen multiresistenten Keim umfassen. Aufgrund der hohen Selektivität, geringen Zytotoxizität in antimikrobiellen Konzentrationen und des breiten Wirkungsspektrums sind die erfindungsgemäßen Peptide für die Verwendung als Breitspektrumantibiotikum in einem weiten Anwendungsbereich von lokalen, systemischen oder äußerlichen therapeutischen Anwendungen und zur Bewältigung bakteriellen Multiresistenzen besonders vorteilhaft.

Es ist auch möglich, das Peptid in Kombination mit einem weiteren antimikrobiellen Wirkstoff zu verwenden. Insbesondere ist die Verwendung in Kombination mit einem Antibiotikum ausgewählt aus der Gruppe der beta-Lactame, Glycopeptide, Polyketide, Aminoglycoside, Polypeptid-Antibiotika, Chinolone, Sulfonamide und Kombinationen davon vorgesehen. Es ist von Vorteil, dass die erfindungsgemäßen Peptide im Vergleich zu den herkömmlichen Antibiotika einen anderen Wirkmechanismus besitzen, sodass sich ein Synergieeffekt der beiden Wirkstoffklassen ergibt, welcher für die Behandlung von bakteriellen Infektionen, insbesondere mit bereits antibiotikaresistenten Keimen, besonders gut geeignet ist. Auf diese Weise können auch solche Antibiotika eine neue medizinische Verwendung finden, gegen welche bereits Resistenzen entwickelt wurden.

In einem weiteren Aspekt der Erfindung ist eine antimikrobielle Zusammensetzung umfassend das oben beschriebene Peptid vorgesehen. Eine antimikrobielle Zusammensetzung kann eine pharmazeutische Zusammensetzung sein. Die pharmazeutische Zusammensetzung enthält vorzugsweise eine therapeutisch effektive Menge des Peptids. Die pharmazeutische Zusammensetzung kann unter anderem Hilfsstoffe enthalten. Solche Hilfsstoffe können beispielsweise Laktose, Cellulose, Stärke, Saccharose, Paraffin, Hartfett, Polyethylenglykol und/oder Polyethylenoxid sowie deren Derivate umfassen. Die pharmazeutische Zusammensetzung kann auch einen Proteasehemmer umfassen. Die pharmazeutische Zusammensetzung kann für die lokale, systemische oder äußerlichen Anwendung vorgesehen sein. Die pharmazeutische Zusammensetzung kann insbesondere auch ein weiteres Antibiotikum wie bereits vorangehend beschrieben enthalten.

In einer anderen Ausführungsform ist die antimikrobielle Zusammensetzung eine Beschichtung, zum Beispiel eine Antifouling-Beschichtung. Solche Beschichtungen können beispielsweise an Grenzflächen eingesetzt werden um die Ansiedlung von Mikroorganismen, sogenannte Biofilme, zu verhindern beziehungsweise zu bekämpfen. In einer Ausführungsform ist die Beschichtung von medizinischen Vorrichtungen vorgesehen. Ein nicht beschränkendes Beispiel hierfür ist die Beschichtung von medizinischen Implantaten, beispielsweise von Endoprothesen, Kathetern, Tubussen oder Stents. In einer anderen Ausgestaltung ist die Beschichtung von wasser- beziehungsweise witterungsausgesetzten Oberflächen vorgesehen, zum Beispiel von Bootskörpern, Rohrleitungen oder Verglasungen. Die Beschichtung kann auch textile Erzeugnisse betreffen, zum Beispiel Kleidung, Operationstücher oder Bettwäsche.

In bestimmten Ausführungsformen weist das Peptid mindestens 90% oder mindestens 95% Sequenzhomologie mit einem der Peptide der SEQ ID NO:1 bis SEQ ID NO:49 auf. Die prozentuale Sequenzübereinstimmung zwischen zwei Peptidsequenzen kann beispielsweise mit Hilfe des Algorithmus "ClustalW" von Thompson et al. (1994) bestimmt werden.

Vorzugsweise wird das Peptid synthetisch hergestellt, beispielsweise in chemischer Festphasensynthese (engl.: solid phase peptide synthesis, SPPS). Besonders bevorzugt ist die chemische Festphasensynthese an einem Syntheseharz, welches einen Amidlinker aufweist. Auf diese Weise liegt das Peptid nach der Abspaltung vom Harz als Säureamid vor, welches besonders gute antimikrobielle Eigenschaften aufweist. Gleichzeitig ist das Peptid durch die Amidierung besser gegen einen proteolytischen Abbau geschützt, was für die Verwendung als Therapeutikum von Vorteil ist. Mit diesem Verfahren kann das Peptid besonders einfach und wirtschaftlich hergestellt werden.

Kurzbeschreibung der Figuren

Figur 1 zeigt ein repräsentatives Auswertungsbeispiel zur Bestimmung der minimalen Hemmkonzentration (MHK). In dem Säulendiagramm ist die Wachstumsinhibierung in Prozent über der entsprechenden Peptidkonzentration in μM beispielhaft anhand der Referenzpeptide Protamin, KL-Helix und dem erfindungsgemäßen Peptid O1 dargestellt.

Figur 2 zeigt exemplarisch einen Dotplot einer durchflusszytometrischen Messung von einer Probe mit etwa 50% lebenden (A) und 50% toten Bakterien (B) sowie Kalibrierpartikeln (C).

Figur 3 zeigt exemplarisch die numerische Auswertung einer durchflusszytometrischen Messung zur Bestimmung der membranpermeabilisierenden Eigenschaften der antimikrobiellen Peptide anhand des erfindungsgemäßen Peptids O1. In dem Säulendiagramm ist die Bakterienkonzentration aufgeschlüsselt in lebende, tote, unbekannte und gesamte Bakterien über der jeweiligen Peptidkonzentration für Escherichia coli (E. coli) und Staphylococcus xylosus (S. xylosus) getrennt aufgetragen.

Figur 4 zeigt eine beispielhafte grafische Auswertung zur kinetischen Untersuchung der Membranpermeabilisierung anhand der Messwerte des erfindungsgemäßen Peptids N4 für die äußere Bakterienmembran.

Figur 5 zeigt beispielhaft die grafische Auswertung zur kinetischen Untersuchung der Membranpermeabilisierung anhand der Messwerte des erfindungsgemäßen Peptids O1 für die äußere Bakterienmembran.

Figur 6 zeigt repräsentative grafische Auswertungen zur Zytotoxizitätsbestimmung anhand der Referenzpeptide BMAP-27, Protamin, KL-Helix und dem erfindungsgemäßen Peptid O1.

Figur 7 zeigt repräsentative Ergebnisse der konzentrations- und peptidabhängigen Membranpermeabilisierung von S. xylosus und der humanen Zelllinie U937 in einem Mischkulturexperiment. In dem Säulendiagramm ist die Zellkonzentration aufgeschlüsselt nach lebenden und toten Zellen über der Konzentration des natürlichen Referenzpeptids Melittin und dem erfindungsgemäßen Peptid O1 sowie der Negativkontrolle als Vergleichsgröße aufgetragen.

Figur 8 zeigt Modelle der räumlichen Struktur der natürlichen Referenzpeptide Melittin, BMAP-27, Protamine, Indolicidine und KL-Helix.

Figur 9 zeigt räumliche Modelle des erfindungsgemäßen Peptids M1 und der Referenzpeptide M2 bis M6.

Figur 10 zeigt Modelle der räumlichen Struktur der erfindungsgemäßen Peptide N1 bis N4 im Vergleich zum erfindungsgemäßen Peptid M1.

Figur 11 zeigt Modelle der räumlichen Struktur der erfindungsgemäßen Peptide C1 bis C4 im Vergleich zum erfindungsgemäßen Peptid M1.

Figur 12 zeigt Modelle der räumlichen Struktur der erfindungsgemäßen Peptide H1 bis H3 und dem Referenzpeptid H8 im Vergleich zum erfindungsgemäßen Peptid M1.

Figur 13 zeigt Modelle der räumlichen Struktur des erfindungsgemäßen Peptids A1 sowie der Referenzpeptide A2 bis A5.

Figur 14 zeigt Modelle der räumlichen Struktur der Referenzpeptide D1 bis D3 und D8 sowie der erfindungsgemäßen Peptide D4 bis D7.

Figur 15 zeigt Modelle der räumlichen Struktur des erfindungsgemäßen Peptids O1 in wässriger Umgebung gemäß Vorhersage mit den PEP-fold Webserver (A), sowie Modelle der räumlichen Struktur des Peptids O1 in einem wässrigen (B) und einem hydrophoben (C) Medium sowie in Vakuum (D) gemäß Vorhersage mit dem PEPstr peptide tertiary structure prediction webserver.

Kurzbeschreibung der Sequenzen

SEQ ID NO:1 bis SEQ ID NO:49 sind erfindungsgemäße Peptide.

SEQ ID NO: 50 bis SEQ ID NO: 79 sind Referenzpeptide.

In den nachfolgenden Beispielen werden bestimmte Ausführungsformen der Erfindung in Anlehnung an Figuren und experimentelle Daten näher beschrieben. Die Beispiele und Figuren dienen nicht der Beschränkung auf bestimmte Details.

Beispiel 1

Antimikrobielle Peptide

Für die Untersuchung wurden natürliche antimikrobielle Referenzpeptide aus verschiedenen Quellen untersucht. Melittin extrahiert aus Honigbienengift wurde in über 85% Reinheit über Sigma-Aldrich (Taufkirchen, Deutschland) bezogen. Protamine aus Lachs wurde in Grad IV Qualität histonfrei ebenfalls über Sigma-Aldrich bezogen. Die Peptide Indolicidine, BMAP-27, das alpha-helikale KL Peptid sowie die Bibliothek der erfindungsgemäßen Peptide und Vergleichspeptide wurden per Auftragssynthese von der JPT Peptide Technologies GmbH (Berlin, Deutschland) bezogen. Soweit im Folgenden keine abweichenden Angaben gemacht werden, wurden die Peptide am C-Terminus als freie Säure (COOH) hergestellt. Die erfindungsgemäßen Peptide können vorteilhafterweise besonders einfach und wirtschaftlich in Festphasensynthese hergestellt werden. Eine Qualitätskontrolle der Peptide wurde mit HPLC/MS Analyse durchgeführt. Für die nachfolgenden Untersuchungen wurden die Peptide in einer Reinheit von mindestens 70% verwendet. Alle Peptide wurden in einem lyophilisierten Zustand bei -20 °C gelagert und erst vor der Verwendung in einer Konzentration von 2 mM in deionisiertem Wasser gelöst.

Beispiel 2

Bestimmung der minimalen Hemmkonzentration (MHK)

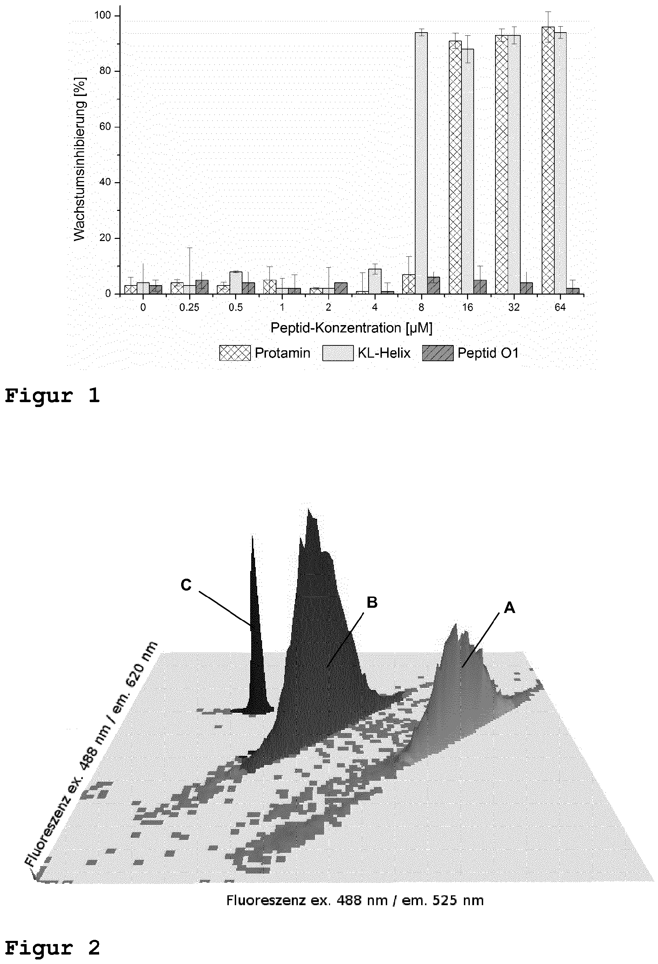

Die minimale Hemmkonzentration (MHK) der Peptide wurde beispielhaft gegen gram(-) E. coli DH5$\alpha$ und gram(+) Staphylococcus xylosus (beides Deutsche Sammlung von Mikroorganismen und Zellkulturen, DSMZ) in einer standardisierten Mikrodilutionsmethode nach den allgemeinen Richtlinien des CLSI (Clinical and Laboratory Standards Institute 2006) und EUCAST (European Committee for Antimicrobial Susceptability Testing of the European Society of Clinical Microbiology and Infectious Diseases 2003) mit geringfügigen Anpassungen an die Untersuchung kationischer antimikrobieller Peptide durchgeführt (Wiegand et al. 2008). Die Bakterienzellen wurden in Mueller-Hinton-Boullion (MHB) bis zu einer optischen Dichte von 0,4 bis 0,6 bei 600 nm Wellenlänge, entsprechend etwa $4 \cdot 10^8$ Bakterien pro ml, kultiviert. Anschließend wurden die Bakterienkulturen auf eine Bakterienkonzentration von etwa $1 \cdot 10^5$ Bakterien pro ml in sterilem MHB verdünnt. Von den Peptiden wurden Reihen von 2-fach Verdünnungen beginnend bei 128 $\mu$M bis zur geringsten Verdünnung von 0,5 $\mu$M in MHB hergestellt. Die Peptidverdünnungen wurden mit den verdünnten Bakterienkulturen in einem 1:2 Volumenverhältnis in den Kammern einer 96-Well Polypropylen-Mikrotiterplatte (Starlab, Hamburg, Deutschland) gemischt, so dass die Peptide in einer Endkonzentration zwischen 64 $\mu$M und 0,25 $\mu$M bei einer Bakterienkonzentration von $5 \cdot 10^4$ Bakterien/ml vorlagen. Steriles MHB diente als Negativkontrolle, als Positivkontrolle diente die entsprechende Bakterienkonzentration in MHB ohne den Zusatz von Peptid. Die Mikrotiterplatten wurden für 18 Stunden bei 37 °C unter Schütteln bei 120 U/min in einer Klimakammer inkubiert. Nach der Inkubation wurde die Bakterienkonzentration durch erneute Absorptionsmessung bei 600 nm Wellenlänge bestimmt. Als MHK wurde die geringste Peptidkonzentration bestimmt, bei der kein Unterschied in der Absorption zwischen der Probe und der Positivkontrolle festgestellt werden konnte. Eine repräsentative Auswertung ist in Figur 1 gezeigt.

Beispiel 3

Durchflusszytometrische Bestimmung der Zellviabilität

Die Zerstörung der Bakterienmembranen durch die antimikrobiellen Peptide wurde durchflusszytometrisch mit Hilfe eines Lebend/Tot-Färbeverfahrens in Kombination mit einem internen Kalibrierungs- und Zählsystem untersucht. Zu diesem Zweck wurde SYBR Green I (Sigma-Aldrich) als Gesamtfärbung und Propidiumiodid (Sigma-Aldrich) als Gegenfärbung für membrangeschädigte Bakterien verwendet. Die Kalibrierung und Quantifizierung des Messverfahrens wurde mit Hilfe von PeakFlow Orange Flow Cytometry Reference Beats (Life Technologies, Darmstadt, Deutschland) erreicht. Die Durchflussanalyse wurde an einen Cytomics FC500 Instrument (Beckmann Coulter, München, Deutschland) ausgestattet mit einem Argonlaser (488 nm) und den entsprechenden Emissionsfiltern für SYBR Green I (525 nm) und Propidiumiodid (620 nm) durchgeführt. Alle Detektoren wurden auf eine logarithmische Skala eingestellt und die Gates wurden durch Vorversuche mit lebenden und Isopropanol-abgetöteten Bakterien vorjustiert. Optisches und elektronisches Rauschen wurde durch die Vorjustierung eines Schwellenwertes auf dem Vorwärtsstreulichtdetektor eliminiert. Die Durchflussrate wurde in etwa im Bereich von 300 Ereignissen pro Sekunde gehalten. Die gesamte Messerfassung betrug 20.000 Ereignisse. Vor der Probenanalyse wurden die Farbstoffe, Kalibrierungspartikel und Trägerflüssigkeit in einem Mastermix kombiniert. Für eine Messung wurden 50 $\mu$l Bakterienkultur zu 450 $\mu$l Mastermix gegeben, so dass Endkonzentrationen von 10 $\mu$M Propidiumiodid, 2-fach SYBR Green I und $1{,}2 \cdot 10^5$ Partikel/ml vorlagen. Danach wurde das Gemisch für 10 min im Dunkeln bei Raumtemperatur inkubiert und anschließend durchflusszytometrisch vermessen. Die Anzahl der Ereignisse in jedem Gate wurde mit der CXP Analyses 2.2 Software erfasst und evaluiert. Die Gesamtzahl der lebenden und toten Bakterien wurde berechnet. Ereignisse, welche weder einer lebenden noch einer toten Bakterienzelle zugeordnet werden konnte, wurden von der Berechnung ausgenommen. Ein beispielhafter Dotplot als Ergebnis der durchflusszytometrischen Vermessung einer Probe mit ca. 50% lebenden Bakterien (A) und 50% toten Bakterien (B) sowie Kalibrierpartikeln (C) ist in Figur 2 gezeigt.

Beispiel 4

Bestimmung der permeabilisierenden Eigenschaften der antimikrobiellen Peptide gegenüber Bakterienmembranen

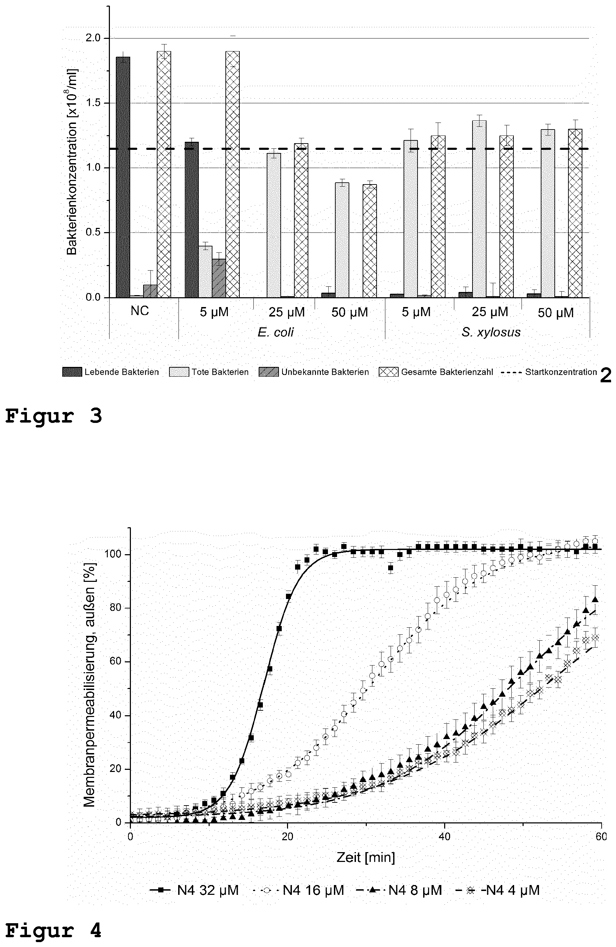

Für die Bestimmung der depolarisierenden Eigenschaften der antimikrobiellen Peptide wurden jeweils drei verschiedene Peptidkonzentrationen beispielhaft gegen gram(-) E. coli DH5$\alpha$ und gram(+) Staphylococcus xylosus getestet. Die Bakterien wurden in MHB kultiviert bis zu einer optischen Dichte von 0,4 bis 0.6 bei 600 nm, entsprechend etwa $4 \cdot 10^8$ Bakterien/ml. Anschließend wurde die Bakterienkonzentration auf etwa $2 \cdot 10^8$ Bakterien/ml in MHB durchflusszytometrisch eingestellt. Die antimikrobiellen Peptide wurden in MHB verdünnt und zur Bakterienkultur gegeben, so dass finale Peptidkonzentrationen von 50 $\mu$M, 25 $\mu$M und 5 $\mu$M in Kombination mit einer finalen Bakterienkonzentration von $1 \cdot 10^8$ Bakterien/ml vorlag. Danach wurden die Proben zunächst für 2 Stunden bei 37 °C unter leichten Schütteln inkubiert. Im Anschluss wurden die Bakterien durchflusszytometrisch wie in Beispiel 3 beschrieben untersucht. Es wurde die niedrigste Peptidkonzentration ermittelt, bei welcher zumindest 80% der Bakterienpopulation permeabilisierte Membranen aufwies. Ein repräsentatives Beispielergebnis ist in Figur 3 für das Peptid O1 gezeigt. In dem Säulendiagramm ist getrennt nach E. coli und S. xylosus jeweils die ermittelte Anzahl von lebenden, toten, unbekannten und gesamten Bakterien in Abhängigkeit von der eingesetzten Peptidkonzentration dargestellt. Als Vergleichsgröße sind die entsprechenden Bakterienkonzentrationen in der Negativkontrolle (NC) gezeigt.

Beispiel 5

Untersuchung der Permeabilisierung der äußeren und inneren Bakterienembran

Die Fähigkeit der antimikrobiellen Peptide zur jeweiligen Permeabilisierung der äußeren und inneren Bakterienembran wurde beispielhaft an E. coli ML-35p (zur Verfügung gestellt von Dr. R.E. Lehrer, Center for Health Sciences, Los Angeles, CA, USA) getestet. Die Peptide wurden in serieller Verdünnung mit E. coli ML-35p inkubiert und die zeitabhängige Freisetzung zellinterner Komponenten in einem geringfügig abgeänderten Protokoll nach Ericsson et al. (2002) gemessen. Zu diesem Zweck wurden die Bakterien in Luria-Bertani (LB) Medium mit 100 $\mu$g/ml Ampicillin (Fluka, Taufkirchen, Deutschland) bis zum Erreichen einer optischen Dichte von 0,4 bei 600 nm Wellenlänge angezogen. Anschließend wurden die Zellen drei Mal durch Zentrifugation bei 4000 g für 1 min und anschließende Wiederaufnahme in 10 mM HEPES Puffer pH 7,2 gewaschen. Abschließend wurden die Zellen auf eine optische Dichte von 0,3 bei 600 nm Wellenlänge verdünnt. Zur Messung der Permeabilisierung der äußeren Membran wurde Nitrocefin (Merck, Darmstadt, Deutschland) in HEPES Puffer auf eine Konzentration von 60 $\mu$g/ml verdünnt. Die antimikrobiellen Peptide wurden in HEPES Puffer auf eine Endkonzentration von 12 $\mu$M, 24 $\mu$M, 48 $\mu$M und 96 $\mu$M Endkonzentration verdünnt. Im

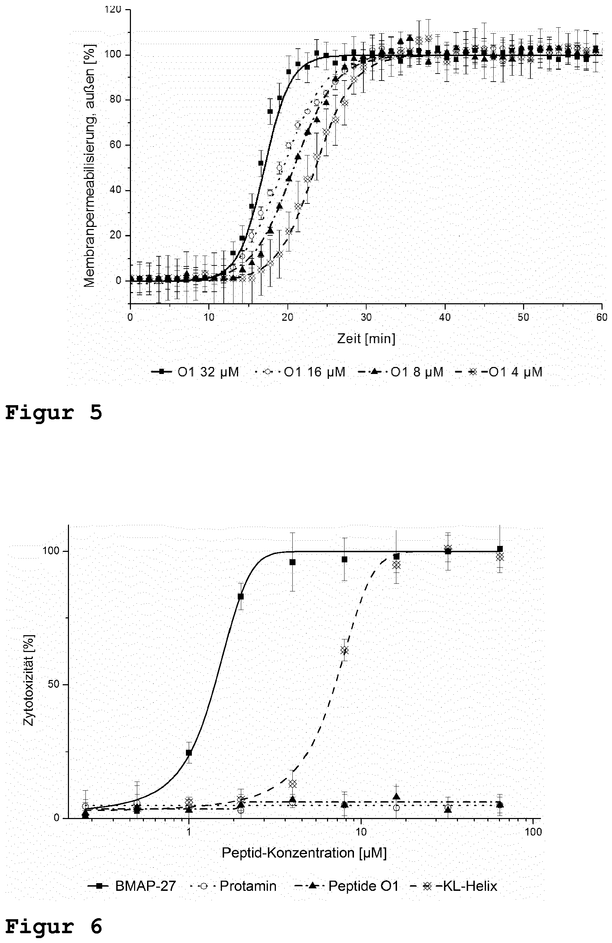

nächsten Schritt wurden 50 μl Nitrocefin mit 50 μl antimikrobiellem Peptid und 50 μl Bakteriensuspension versetzt, so dass eine Endkonzentration von 20 μg/ml Nitrocefin, 4 μM bis 32 μM antimikrobielles Peptid und Bakterien mit einer optischen Dichte von 0,1 bei 600 nm vorlagen. Die Proben wurden durchmischt und die Absorption bei 500 nm Wellenlänge wurde unmittelbar über eine Zeitspanne von mindestens 90 min bei 37 °C unter regelmäßigem Schütteln in einem FLUOstar omega Microplate Reader (BMG Labtech, Ortenberg, Deutschland) gemessen. Die Untersuchung der Permeabilisierung der inneren Membran wurde in der gleichen Weise durchgeführt, wobei anstelle des Nitrocefins entsprechend O-Nitrophenyl-beta-D-Galactosid (ONPG, Sigma-Aldrich) in einer Endkonzentration von 100 μg/ml verwendet und die Absorptionsänderung bei 420 nm Wellenlänge bestimmt wurde. Als Kontrolle dienten Proben ohne antimikrobielles Peptid, als Positivkontrolle für die Membranpermeabilisierung diente Polymyxin B (Sigma-Aldrich) in einer Endkonzentration von 5 μM. Für die Datenauswertung wurden von den Messwerten der Proben zunächst die Messwerte der entsprechenden Negativkontrollen abzogen und die Differenz anschließend durch die entsprechenden Messwerte der Permeabilisierungskontrolle geteilt. Anschließend erfolgte eine grafische Auswertung, wobei die berechneten Werte über der Peptidkonzentration aufgetragen und mit Hilfe der Origin Pro 8.1 G Software (OriginLab) nach einem sigmoidalen Modell gefittet wurden. Als repräsentative Beispiele zeigen die Figuren 4 und 5 die grafische Auswertung zur kinetischen Untersuchung der Permeabilisierung der äußeren E. coli Bakterienmembran für die Peptide N4 (Figur 4) und O1 (Figur 5) .

Als Vergleichsgröße zwischen den Peptiden wurden hieraus die Zeitpunkte ermittelt, bei denen 50% des endgültigen Signalniveaus erreicht wurden. Zu diesem Zweck wurde die Gleichung mit der prozentualen Membranpermeabilisierung y, der Zeit x in Minuten, dem unteren Plateau A1, dem oberen Plateau A2, dem Wendepunkt LogX0 und der Hill-Steigung p

$$y = A1 + \frac{A2-A1}{1+10^{(LogX0-x)*p}} \qquad (5.1)$$

verwendet. Die Berechnung der Parameter A1, A2, LogX0 und p wurde durch das Programm anhand der Ausgleichskurve vorgenommen. Die weitere Auflösung der Gleichung 5.1 für den Fall y = 50 lieferte dann für x den gesuchten Zeitpunkt, bei dem das halbmaximale Signalniveau erreicht wurde.

Beispiel 6

Berechnung der Zytotoxizität und des therapeutischen Verhältnisses der antimikrobiellen Peptide

Das zytotoxische Potential der antimikrobiellen Peptide wurde beispielhaft anhand der humanen histiozytären Lymphomzelllinie U937 (DSMZ) bestimmt. Der Einfluss der Peptide auf die Permeabilität der Zellmembran sowie ihre längerfristige wachstumshindernde Wirkung wurde untersucht. 15 ml RPMI 1640 Medium mit 2 mM Glutamin und 10% FCS (Biochrom, Berlin, Germany) wurde mit $1 \cdot 10^5$ Zellen pro ml inokuliert und für drei Tage bei 37° in T75-Zellkulturflaschen (TPP, Trasadingen, Schweiz) unter konstanter Begasung mit 5% $CO_2$ in einer Klimakammer kultiviert. Das Medium wurde mit 10 U/ml Penicillin und 100 mg/ml Streptomycin (beides AppliChem, Darmstadt, Deutschland) zur Vermeidung von bakteriellen Kontaminationen versetzt, wobei während der nachfolgenden Rekultivierung keine weiteren Antibiotika zugesetzt wurden. Vor jedem Experiment wurden die Zellen durch Zentrifugation bei 100 g für fünf Minuten und anschließende Resuspendierung in RPMI 1640 Medium mit 2 mM Glutamin und 10% FCS jeweils zwei Mal gewaschen und auf eine Konzentration von $1 \cdot 10^5$ Zellen pro ml eingestellt. Zum Schluss wurden je 100 μl der U937-Zellen in die Reaktionskammern einer sterilen 96-Well Polypropylen-Mikrotiterplatte überführt und mit je 100 μl eines vorverdünnten antimikrobiellen Peptids in RPMI 1640 Medium ohne Antibiotikazusatz versetzt, sodass Endkonzentrationen von je $5 \cdot 10^4$ Zellen pro ml bei Peptidkonzentrationen zwischen 0,25 μM und 64 μM vorlagen. Als Negativkontrolle dienten Proben ohne antimikrobielles Peptid, während Triton X-100 (AppliChem) in einer Endkonzentration von 5% als Positivkontrolle für die Permeabilisierung eingesetzt wurde. Die Proben wurden für zwei Stunden bei 37 °C und kontinuierlicher Begasung mit 5% $CO_2$ in einer Klimakammer kultiviert. Anschließend wurden je 100 μl von jeder Probe abgenommen und in neue sterile Mikrotiterplatten zur Bestimmung der direkten Membranschädigung überführt. Die restlichen 100 μl der Proben wurden für mindestens vier weitere Tage kultiviert.

Die direkte Membranschädigung wurde mit Hilfe des CytoTox 96 Non-Radioactive Cytotoxicity Assay (Promega, Mannheim, Deutschland) untersucht. Zu diesem Zweck wurden die Zellen bei 100 g für zehn Minuten bei 4 °C sedimentiert und 50 μl des resultierenden Kulturüberstandes zu 50 μl des CytoTox-Substratmix gegeben. Nach 30 Minuten Inkubationszeit im Dunkeln bei Raumtemperatur wurden 50 μl der CytoTox-Stopplösung zur Probe gegeben und die Absorption bei 490 nm gemessen. Für die Bestimmung des $LD_{50}$-Wertes wurde von den Absorptionswerten der Messwert der Negativkontrolle abgezogen und durch den Messwert der Permeabilisierungskontrolle geteilt. Die berechneten Werte wurden grafisch über der Peptidkonzentration aufgetragen und mit der Origin pro 8.1 G Software nach einem sigmoidalen Modell gefittet und hieraus die $LD_{50}$-Werte ermittelt. Die Berechnung erfolgte analog zum Beispiel 5 anhand der Lösung der Gleichung 5.1 für den Fall y = 50, wobei y in diesem Fall der relativen Zytotoxizität in Prozent und x der Peptidkon-

zentration in $\mu$M entspricht. Repräsentative grafische Auswertungen von LD$_{50}$-Werten sind in Figur 6 anhand der Messergebnisse für die Referenzpeptide BMAP-27, Protamin und KL-Helix sowie das erfindungsgemäße Peptid O1 gezeigt.

Das therapeutische Verhältnis entspricht dem Quotienten aus dem LD$_{50}$-Wert und dem MHK-Wert eines Peptids. In denjenigen Fällen, in denen der LD$_{50}$-Wert oberhalb der gemessenen Peptidkonzentrationen lag, wurde eine LD$_{50}$ von 128 $\mu$M für die Berechnung des therapeutischen Verhältnisses zu Grunde gelegt.

Ein hohes therapeutisches Verhältnis ist demnach vorteilhaft, weil es einer hohen antimikrobiellen Aktivität bei gleichzeitig geringen zytotoxischen Nebeneffekten des antimikrobiellen Peptids entspricht.

Für die Bestimmung der langzeitlichen Wachstumsinhibierung der antimikrobiellen Peptide wurden die Gesamtzellzahlen in den Proben nach Ablauf der weiteren vier Tage Kultivierungszeit bestimmt. Zu diesem Zweck wurden 10 $\mu$l Lysierungslösung zu jeweils 100 $\mu$l der Zellsuspension gegeben und für 60 Minuten bei 37 °C inkubiert. Anschließend wurden die Zellkulturüberstände wie oben beschrieben untersucht. Die niedrigste Peptidkonzentration, bei der eine vollständige Unterdrückung des Zellwachstums nach vier Tagen festgestellt wurde, wurde als minimale toxische Konzentration (MTC) festgelegt.

Beispiel 7

Behandlung von bakteriell kontaminierten Zellkulturen

Die Einsatzfähigkeit der antimikrobiellen Peptide als therapeutischer Wirkstoff wurde anhand ihrer selektiven antimikrobiellen Eigenschaften in modellhaften Mischkulturexperimenten überprüft. Zu diesem Zweck wurden die humane Zelllinie U937 und Staphylococcus xylosus wie in Beispiel 6 beziehungsweise Beispiel 2 beschrieben vorbereitet. Anschließend wurde eine Mischkultur mit $1 \cdot 10^6$ Bakterienzellen/ml und $1 \cdot 10^6$ U937-Zellen/ml sowie entweder 25 $\mu$M oder 50 $\mu$M antimikrobiellen Peptids in RPMI 1640 Medium hergestellt. Steriles Medium diente als Negativkontrolle, während das antimikrobielle Peptid Melittin als Toxizitätskontrolle verwendet wurde. Die Mischkulturen wurden bei konstanter Begasung mit 5% $CO_2$ in einer Klimakammer für zwei Stunden bei 37 °C inkubiert. Anschließend wurden die Mischzellkulturen gefärbt und durchflusszytometrisch wie in Beispiel 3 beschrieben vermessen, wobei die Messskala an die Vermessung der größeren eukaryotischen Zellen angepasst wurde.

Beispiel 8

Berechnung der physikalisch-chemischen Peptideigenschaften und Vorhersage der Peptidstruktur

Die Hydrophobizität (H) und das hydrophobe Moment ($\mu$) sowie die Helixrad-Projektion wurden unter Annahme einer idealen alphahelikalen Konformation für jedes individuelle antimikrobielle Peptid berechnet. Die Berechnung wurde mit Hilfe des HeliQuest Webservers (Gautier et al. 2008) unter Annahme eines neutralen Mediums bei pH 7,4 durchgeführt. Die wahrscheinlichste dreidimensionale Struktur der Peptide wurde unter der Annahme eines wässrigen Mediums mit Hilfe des PEPFOLD Webservers berechnet (Thevenet et al. 2012, Maupetit et al. 2010, Maupetit et al. 2009). Zusätzlich wurden die ermittelten dreidimensionalen Strukturen mit Hilfe des PEPstr Peptide Tertiary Structure Prediction Webservers bestätigt (Kaur et al. 2007), wobei für die Berechnungen ein Vakuum, ein wässriges sowie ein hydrophobes Medium zugrunde gelegt wurde. Peptide mit weniger als neun Aminosäuren wurden von der Berechnung ausgenommen. Die Strukturvorhersagen wurden mit der Geneious 6.1.5 Software verarbeitet (www.geneious.com) und mithilfe der UCSF Chimera Software visualisiert (Pettersen et al. 2004). Die grafische Darstellung erfolgte im Ribbon Style Model, wobei die alpha-Helices als Windungen und die beta-Stränge als Pfeile dargestellt sind. Die Pfeilspitzen zeigen zum C-Terminus.

Beispiel 8

Antimikrobielle Eigenschaften natürlicher Referenzpeptide

Als Vergleichsgröße für die antimikrobiellen Eigenschaften der isolierten Peptide der vorliegenden Erfindung wurden die antimikrobiellen Eigenschaften der vier natürlichen AMPs Melittin (SEQ ID NO:50), BMAP-27 (SEQ ID NO:51), Protamine (SEQ ID NO:52) und Indolicidine (SEQ ID NO:53) sowie des nicht natürlich vorkommenden alpha-Helix formenden KL-Peptids (SEQ ID NO:54) untersucht. Die Ergebnisse sind in Tabelle 1 zusammengefasst.

**Tabelle 1:** Zusammenfassung der physikalisch-chemischen, antimikrobiellen und zytotoxischen Eigenschaften der natürlichen Referenzpeptide. Alle Werte repräsentieren das arithmetische Mittel der Ergebnisse aus vier unabhängigen Testreihen. [1]Sequenzangabe im Einbuchstabencode vom N-Terminus zum C-Terminus. [2]Werte in Klammern bedeuten, dass zwar eine MTC aber keine LD$_{50}$ festgestellt wurde.

| Peptid | Sequenz[1] | Eigenschaften | | MHK [μM] | | Toxizität [μM] | | Therapeutisches Verhältnis[2] | |
|---|---|---|---|---|---|---|---|---|---|
| | | H | μ | E. coli | S. xylosus | LD$_{50}$ | MTC | E. coli | S. xylosus |
| Melittin | GIGAVLKVLTTGLPALISWIKRKRQQ | 0.511 | 0.394 | 8 | 2 | 1.4 | 2.0 | 0.18 | 0.7 |
| BMAP-27 | GRFKRFRKKFKKLFKKLSPVIPLLHLG | 0.380 | 0.456 | 1 | 2 | 1.8 | 4.0 | 1.80 | 0.9 |
| Protamine | MPRRRRSSSRPVRRRRRPRVSRRR RRRGGRRR | 0.454 | 0.086 | 8 | 0.5 | >64 | 16 | (16) | (256) |
| Indolicidine | ILPWKWPWWPWRR | 1.069 | 0.190 | 4 | 32 | >64 | >64 | 32 | 4 |
| KL-Helix | LKLLKKLLKKLLKLL | 0.624 | 0.836 | 4 | 2 | 7.0 | 8.0 | 1.75 | 3.5 |

Die Referenzpeptide weisen eine generelle antimikrobielle Aktivität gegenüber E. coli und S. xylosus auf, wobei die MHK-Werte zwischen 1 μM und 8 μM beziehungsweise 0,5 μM und 32 μM liegen. Gleichzeitig weisen die Peptide Melittin, BMAP-27 und KL-Helix starke zytotoxische Eigenschaften gegenüber der Modellzelllinie U937 auf, wobei die $LD_{50}$-Werte zwischen 1,4 μM und 7 μM liegen. Da diese Werte im Wesentlichen den jeweiligen MHK-Werten entsprechen, liegt ein unvorteilhaftes therapeutisches Verhältnis von ungefähr 1 vor. Lediglich Protamine und Indolicidine weisen aufgrund geringer zytotoxischer Eigenschaften vorteilhaftere therapeutische Verhältnisse auf, wobei Protamine jedoch eine bedenkliche vollständige Wachstumsinhibierung von eukaryotischen Zellen bereits bei einer Konzentration von 16 μM zeigt.

Tabelle 2 zeigt die entsprechenden membranzerstörenden Eigenschaften der Referenzpeptide. Während Melittin, BMAP-27 und KL-Helix die Bakterienmembranen innerhalb weniger Minuten zerstören, lassen sich bei Indolicidine und Protamine nur geringe oder gar keine Membranzerstörung nachweisen.

**Tabelle 2:** Zusammenfassung der membranzerstörenden Eigenschaften der natürlichen Referenzpeptide. Alle Werte repräsentieren das arithmetische Mittel der Ergebnisse aus vier unabhängigen Testreihen.

| Peptid | Allgemeine Permeabilisierung [μM] | | Äußere Membran [min] | | | | Inere Membran [min] | | | |
|---|---|---|---|---|---|---|---|---|---|---|
| | *E. coli* | *S. xylosus* | 4 μM | 8 μM | 16 μM | 32 μM | 4 μM | 8 μM | 16 μM | 32 μM |
| Melittin | 25 | 5 | 14 | 9 | 2 | <1 | 18 | 12 | 9 | 6 |
| BMAP-27 | 5 | 5 | <1 | <1 | <1 | <1 | 16 | 9 | 7 | 5 |
| Protamine | >50 | >50 | <1 | <1 | <1 | <1 | >90 | >90 | >90 | >90 |
| Indolicidine | >50 | 50 | >90 | >90 | >90 | >90 | >90 | >90 | >90 | >90 |
| KL-Helix | 5 | 5 | 2 | <1 | <1 | <1 | 14 | 11 | 9 | 6 |

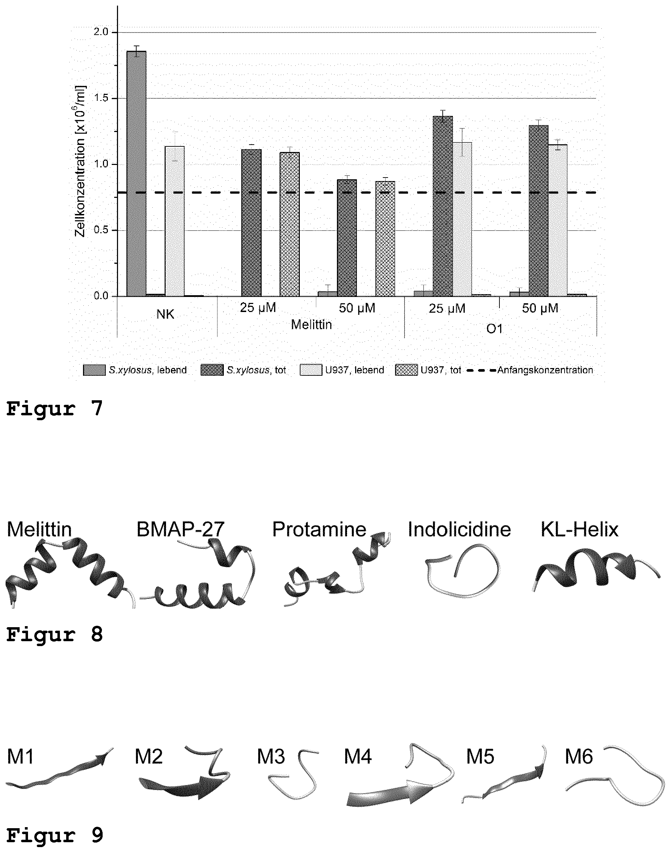

Die Peptide weisen auch in Bezug auf ihre physikalisch chemischen Eigenschaften und dreidimensionale Strukturen Unterschiede auf (Figur 8). Melittin, BMAP-27, Protamine und KL-Helix bilden ausgedehnte alphahelikale Bereiche, während Protamine zwei kurze terminale alphahelikale Bereiche und einen großen zentralen unstrukturierten Bereich aufweist. Indolicidine weist hingegen eine weniger definierte Struktur auf, welche in einer Random Coil Konformation resultiert. Die alphahelikalen Peptide Melittin, BMAP-27 und KL-Helix weisen eine Hydrophobizität zwischen 0,38 und 0,624 sowie ein hydrophobes Moment zwischen 0,394 und 0,836 auf. Lediglich Indolicidine hat mit 1,069 eine höhere Hydrophobizität als die alphahelikalen Peptide. Gleichzeitig mangelt es Indolicidine an Amphipatizität, was aus dem geringen hydrophoben Moment von 0,19 zu erkennen ist. Demgegenüber hat das weitgehend unstruktierte Protamin weder hohe Hydrophobizität noch hohe Amphipatizität.

Beispiel 9

Ausführung des allgemeinen Strukturmotivs

Die antimikrobielle Wirkung des erfindungsgemäßen peptidischen Strukturmotivs $A_xB_yC_z$ wurde beispielhaft anhand eines repräsentativen erfindungsgemäßen Peptids M1 (SEQ ID NO:1) sowie fünf Referenzpeptiden M2 (SEQ ID NO:55), M3 (SEQ ID NO:56), M4 (SEQ ID NO:57), M5 (SEQ ID NO:59) bis M6 (SEQ ID NO:59) untersucht, wobei die Referenzpeptide jeweils im aminoterminalen Bereich A, carboxyterminalen Bereich C oder im dazwischen liegenden Bereich B von der erfindungsgemäßen Peptidstruktur abweichen (Tabelle 3).

**Tabelle 3:** Zusammenfassung der physikalisch-chemischen, antimikrobiellen und zytotoxischen Eigenschaften der Peptide M1 bis M6. Alle Werte repräsentieren das arithmetische Mittel der Ergebnisse aus vier unabhängigen Testreihen. [1]Sequenzangabe im Einbuchstabencode vom N-Terminus zum C-Terminus.

| Peptid | Sequenz[1] | Eigenschaften | | MHK [μM] | | Toxizität [μM] | | Therapeutisches Verhältnis | |
|---|---|---|---|---|---|---|---|---|---|
| | | H | μ | *E. coli* | *S. xylosus* | $LD_{50}$ | MTC | *E. coli* | *S. xylosus* |
| M1 | KRKILILIKRK | 0.256 | 0.290 | 8 | 2 | >64 | >64 | 16 | 64 |

(fortgesetzt)

| Peptid | Sequenz[1] | Eigenschaften | | MHK [$\mu$M] | | Toxizität [$\mu$M] | | Therapeutisches Verhältnis | |
|---|---|---|---|---|---|---|---|---|---|
| | | H | $\mu$ | E. coli | S. xylosus | LD$_{50}$ | MTC | E. coli | S. xylosus |
| M2 | KRKILILILIL | 1.001 | 0.194 | >64 | >64 | >64 | >64 | 1 | 1 |
| M3 | ILILILILKRK | 1.001 | 0.194 | >64 | >64 | >64 | >64 | 1 | 1 |
| M4 | KRKILILIGSG | 0.525 | 0.249 | >64 | >64 | >64 | >64 | 1 | 1 |
| M5 | GSGILILIKRK | 0.525 | 0.249 | >64 | >64 | >64 | >64 | 1 | 1 |
| M6 | KRKSGSGSKRK | -0.555 | 0.090 | >64 | >64 | >64 | >64 | 1 | 1 |

Es wird deutlich, dass das Peptid M1 eine intensive antimikrobielle Wirkung aufweist, wobei die MHK-Werte bei 8 $\mu$M für E. coli und 2 $\mu$M für S. xylosus liegen. Gleichzeitig konnten beim Peptid M1 keine nachteiligen zytotoxischen Eigenschaften festgestellt werden.

Aus der Tabelle 4 geht hervor, dass das Peptid M1 ab einer Konzentration von 25 $\mu$M gegenüber den Bakterien allgemeine Membran zerstörenden Eigenschaften aufweist. Die äußere Bakterienmembran wird durch das Peptid der getesteten Konzentration innerhalb von 16 bis 39 min permeabilisiert, während die Permeabilisierung der inneren Membran eine Konzentration von 16 $\mu$M erfordert und etwa 30 min später eintritt.

**Tabelle 4:** Zusammenfassung der membranzerstörenden Eigenschaften der Peptide M1 bis M6. Alle Werte repräsentieren das arithmetische Mittel der Ergebnisse aus vier unabhängigen Testreihen.

| Peptid | Allgemeine Permeabilisierung [$\mu$M] | | Äußere Menbran [min] | | | | Innere Menbran [min] | | | |
|---|---|---|---|---|---|---|---|---|---|---|
| | E. coli | S. xylosus | 4 $\mu$M | 8 $\mu$M | 16 $\mu$M | 32 $\mu$M | 4 $\mu$M | 8 $\mu$M | 16 $\mu$M | 32 $\mu$M |
| M1 | 25 | 25 | 39 | 16 | 17 | 17 | >90 | >90 | 59 | 42 |
| M2 | >50 | >50 | >90 | >90 | 23 | 26 | >90 | >90 | >90 | >90 |
| M3 | >50 | >50 | >90 | >90 | 42 | 33 | >90 | >90 | >90 | >90 |
| M4 | >50 | >50 | >90 | >90 | >90 | >90 | >90 | >90 | >90 | >90 |
| M5 | >50 | >50 | >90 | >90 | >90 | >90 | >90 | >90 | >90 | >90 |
| M6 | >50 | >50 | >90 | >90 | >90 | >90 | >90 | >90 | >90 | >90 |

Die Vergleichspeptide M2 und M3, welchen jeweils der Bereich C beziehungsweise A fehlt, zeigen mit MHK-Werten von >64 $\mu$M praktisch keine Wachstumsinhibierung der Bakterienkulturen und weisen ein therapeutisches Verhältnis von 1 auf. Bei diesen Peptiden wird nur eine Permeabilisierung der äußeren Bakterienmembran bei den höchsten getesteten Peptidkonzentrationen beobachtet. Die innere Membran wird nicht zerstört. Bei den Referenzpeptiden M4 bis M6 wird keinerlei antimikrobielle oder zytotoxische Wirkung beobachtet.

Ein Zusammenhang zwischen der antimikrobiellen Wirkung und der Sekundärstruktur der Peptide wird aus Figur 9 deutlich. Das Modellpeptid M1 bildet einen gestreckten beta-Strang aus, welcher nahezu die gesamte Peptidsequenz umfasst. Dagegen bilden die Referenzpeptide M2, M4 und M5 deutlich kürzere beta-Stränge aus, wobei die übrigen Bereiche keine erkennbare Sekundärstruktur aufweisen. Die Referenzpeptide M3 und M6 liegen vollständig als Random Coil vor.

Beispiel 10

Ausführung des aminoterminalen Bereichs A

Die Bedeutung des aminoterminalen Bereichs A wurde mit Hilfe der erfindungsgemäßen Peptide N1 (SEQ ID NO:5), N2 (SEQ ID NO:3), N3 (SEQ ID NO:4) bis N4 (SEQ ID NO:2) sowie der Referenzpeptide N5 (SEQ ID NO:60), N6 (SEQ ID NO:61), N7 (SEQ ID NO:62) bis N8 (SEQ ID NO:63) beispielhaft untersucht, wobei die Anzahl der basischen Aminosäuren im Bereich A von fünf (Peptid N1) bis auf null (Peptide N5 bis N8) reduziert wurde. Gleichzeitig wurde in den Peptiden N6, N7 und N8 ein möglicher Nebeneinfluss des Verhältnisses der Anzahl von Aminosäuren in den Bereichen B und C in Abwesenheit des Bereiches A untersucht. Die Untersuchungsergebnisse sind in den Tabellen 5 und 6 zusammengefasst.

**Tabelle 5:** Zusammenfassung der physikalisch-chemischen, antimikrobiellen und zytotoxischen Eigenschaften der Peptide N1 bis N6 im Vergleich zu M1. Alle Werte repräsentieren das arithmetische Mittel der Ergebnisse aus vier unabhängigen Testreihen. [1]Sequenzangabe im Einbuchstabencode vom N-Terminus zum C-Terminus. [2]t.s. kennzeichnet Peptide mit unzureichender Länge für eine akkurate Strukturvorhersage.

| Peptid | Sequenz[1] | Eigenschaften[2] | | MHK [$\mu$M] | | Toxizität [$\mu$M] | | Therapeutisches Verhältnis | |
|---|---|---|---|---|---|---|---|---|---|
| | | H | $\mu$ | *E. coli* | *S. xylosus* | LD$_{50}$ | MTC | *E. coli* | *S. xylosus* |
| N1 | KRKRKILILIKRK | 0.063 | 0.294 | 8 | 0.5 | 36 | 32 | 4.5 | 72 |
| N2 | RKRKILILIKRK | 0.151 | 0.236 | 8 | 2 | >64 | >64 | 16 | 64 |
| M1 | KRKILILIKRK | 0.256 | 0.290 | 8 | 2 | >64 | >64 | 16 | 64 |
| N3 | RKILILIKRK | 0.381 | 0.400 | 16 | 4 | >64 | >64 | 8 | 32 |
| N4 | KILILIKRK | 0.536 | 0.381 | 32 | 16 | 58 | >64 | 1.8 | 3.6 |
| N5 | ILILIKRK | t.s. | t.s. | 64 | 64 | >64 | >64 | 2 | 2 |
| N6 | LILIKRK | t.s. | t.s. | >64 | >64 | >64 | >64 | 1 | 1 |
| N7 | ILIKRK | t.s. | t.s. | >64 | >64 | >64 | >64 | 1 | 1 |
| N8 | LIKRK | t.s. | t.s. | >64 | >64 | >64 | >64 | 1 | 1 |

Es wird deutlich, dass bereits eine basische Aminosäure im N-terminalen Bereich A (x = 1) der erfindungsgemäßen Peptide ausreicht, um eine starke antimikrobielle Wirkung zu entfalten. Beispielhaft hierfür sind die MHK-Werte von 32 μM und 16 μM gegenüber E. coli und S. xylosus des Peptids N4. Gleichzeitig kann durch weitere Ergänzung auf zum Beispiel x = 2, 3, 4 oder 5 basische Aminosäuren im Bereich A eine weitere Verringerung der minimalen Hemmkonzentration, d.h. eine Verstärkung der antimikrobiellen Wirkung, erreicht werden. Entsprechend weist das Peptid N1 als Beispiel für ein erfindungsgemäßes Peptid mit fünf basischen Aminosäuren im Bereich A vorteilhafte MHK-Werte von 8 μM und 0,5 μM auf. Auf diese Weise können besonders vorteilhafte Peptide mit hoher antimikrobieller Aktivität bereitgestellt werden. Erst die vollständige Eliminierung (x = 0) des Bereiches A im Vergleichspeptid N5 steigert den MHK-Wert auf 64 μM, wohingegen die zusätzliche Reduktion des Bereiches B in den Vergleichspeptiden N6 bis N8 zu MHK-Werten von über 64 μM führt.

Aus Tabelle 6 ist ersichtlich, dass die beobachteten antimikrobiellen Eigenschaften der Peptide mit den Membran zerstörenden Eigenschaften korrelieren. Entsprechend wird bereits bei Vorhandensein einer basischen Aminosäure im aminoterminalen Bereich A ab Konzentrationen von 50 μM und 25 μM eine allgemeine Membranpermeabilisierung bei E. coli beziehungsweise S. xylosus durch die erfindungsgemäßen Peptide beobachtet. Durch zusätzliche basische Aminosäuren im Bereich A tritt die Membranzerstörung bereits bei niedrigeren Peptidkonzentrationen ein. Beispielsweise kann mit dem Peptid N1 schon ab Konzentrationen von 25 μM und 5 μM eine allgemeine Membranzerstörung erreicht werden.

**Tabelle 6:** Zusammenfassung der membranzerstörenden Eigenschaften der Peptide N1 bis N6 im Vergleich mit M1. Alle Werte repräsentieren das arithmetische Mittel der Ergebnisse aus vier unabhängigen Testreihen.

| Peptid | Allgemeine Permeabilisierung [μM] | | Äußere Menbran [min] | | | | Innere Menbran [min] | | | |
|---|---|---|---|---|---|---|---|---|---|---|
| | *E. coli* | *S. xylosus* | 4 μM | 8 μM | 16 μM | 32 μM | 4 μM | 8 μM | 16 μM | 32 μM |
| N1 | 25 | 5 | 21 | 14 | 12 | 11 | >90 | >90 | 38 | 32 |
| N2 | 25 | 5 | 20 | 13 | 12 | 12 | >90 | >90 | 40 | 32 |
| M1 | 25 | 25 | 39 | 16 | 17 | 17 | >90 | >90 | 59 | 42 |
| N3 | 25 | 25 | 41 | 40 | 21 | 13 | >90 | >90 | >90 | 56 |
| N4 | 50 | 25 | 54 | 49 | 31 | 17 | >90 | >90 | >90 | >90 |
| N5 | >50 | >50 | >90 | >90 | 48 | 37 | >90 | >90 | >90 | >90 |
| N6 | >50 | >50 | >90 | >90 | >90 | >90 | >90 | >90 | >90 | >90 |
| N7 | >50 | >50 | >90 | >90 | >90 | >90 | >90 | >90 | >90 | >90 |
| N8 | >50 | >50 | >90 | >90 | >90 | >90 | >90 | >90 | >90 | >90 |

Diese Eigenschaften spiegeln sich auch in den kinetischen Untersuchungen zur Permeabilisierung der äußeren und inneren E. coli Membran wieder. Alle modellhaften Peptide, deren aminoterminaler Bereich A zumindest eine basische Aminosäure aufweist, sind der Lage, die äußeren und innere Membran zu zerstören, wobei bei Peptiden mit einem verkürzten aminoterminalen Bereich eine wirksame Permeabilisierung durch höhere Peptidkonzentrationen oder längere Einwirkzeiträume erreicht werden kann. Bei den Referenzpeptiden N5 bis N8 konnte dagegen keine Membranzerstörung festgestellt werden.

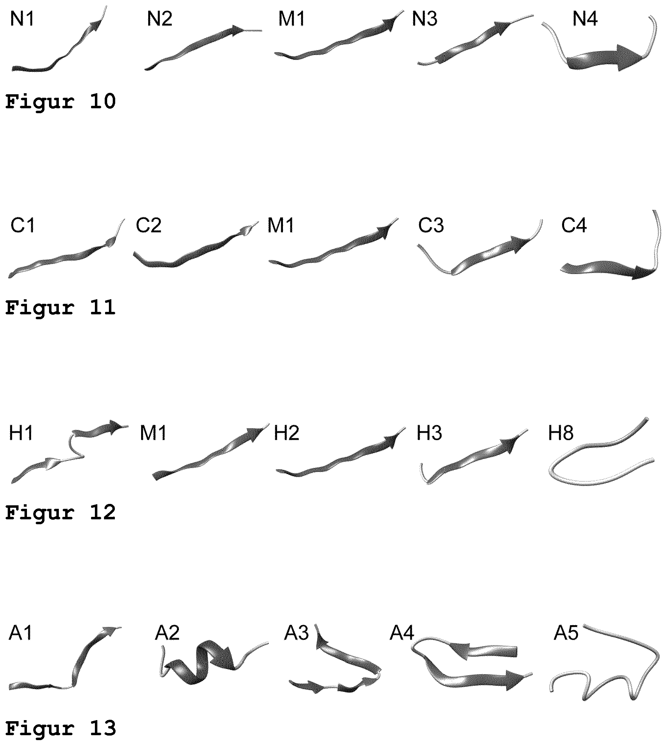

Aus der Figur 10 geht erneut hervor, dass ein enger Zusammenhang zwischen der Entfaltung der antimikrobiellen Wirkung der Peptide und ihrer Sekundärstruktur besteht. Zum Beispiel liegen die Peptide N1 und N2 mit jeweils fünf beziehungsweise vier basischen Aminosäuren im aminoterminalen Bereich A ebenso wie das vorherige Modellpeptid M1 mit drei basischen Aminosäuren im aminoterminalen Bereich A im Wesentlichen über die Gesamtsequenz als beta-Strang vor. Erst die weitere Reduzierung der Anzahl der basischen Aminosäuren im aminoterminalen Bereich A, beispielsweise auf x = 2 beim Peptid N3, führt zur Ausbildung eines unstrukturierten N-terminalen Bereichs. Das Peptid N4 mit nur einer basischen Aminosäure im Bereich A weist noch im zentralen Bereich einen strukturierten beta-Strang auf.

Beispiel 11

Ausführung des carboxyterminalen Bereichs C

Analog zur N-terminalen Region A wurde auch der Einfluss der Anzahl der basischen Aminosäuren im carboxyterminalen Bereich C betrachtet. Die Ergebnisse sind beispielhaft anhand der erfindungsgemäßen Peptide C1 (SEQ ID NO:6), C2 (SEQ ID NO:7), C3 (SEQ ID NO:8) bis C4 (SEQ ID NO:9) sowie der Referenzpeptide C5 (SEQ ID NO:64), C6 (SEQ ID NO:65), C7 (SEQ ID NO:66) bis C8 (SEQ ID NO:67) in Tabelle 7 zusammengefasst.

**Tabelle 7:** Zusammenfassung der physikalisch-chemischen, antimikrobiellen und zytotoxischen Eigenschaften der Peptide C1 bis C8 im Vergleich zu M1. Alle Werte repräsentieren das arithmetische Mittel der Ergebnisse aus vier unabhängigen Testreihen. [1]Sequenzangabe im Einbuchstabencode vom N-Terminus zum C-Terminus. [2]t.s. kennzeichnet Peptide mit unzureichender Länge für eine akkurate Strukturvorhersage.

| Peptid | Sequenz[1] | Eigenschaften[2] | | MHK [$\mu$M] | | Toxizität [$\mu$M] | | Therapeutisches Verhältnis | |
|--------|-----------|------|------|---------|------------|---------|-----|---------|------------|
| | | H | $\mu$ | *E. coli* | *S. xylosus* | $LD_{50}$ | MTC | *E. coli* | *S. xylosus* |
| C1 | KRKILILIKRKRK | 0.063 | 0.294 | 16 | 1 | 61 | >64 | 3.8 | 61 |
| C2 | KRKILILIKRKR | 0.151 | 0.236 | 16 | 2 | >64 | >64 | 8 | 64 |
| M1 | KRKILILIKRK | 0.256 | 0.290 | 8 | 2 | >64 | >64 | 16 | 64 |
| C3 | KRKILILIKR | 0.381 | 0.400 | 16 | 8 | >64 | >64 | 8 | 16 |
| C4 | KRKILILIK | 0.536 | 0.381 | 32 | 8 | >64 | >64 | 4 | 16 |
| C5 | KRKILILI | t.s. | t.s. | >64 | >64 | >64 | >64 | 1 | 1 |
| C6 | KRKILIL | t.s. | t.s. | >64 | >64 | >64 | >64 | 1 | 1 |
| C7 | KRKILI | t.s. | t.s. | >64 | >64 | >64 | >64 | 1 | 1 |
| C8 | KRKIL | t.s. | t.s. | >64 | >64 | >64 | >64 | 1 | 1 |

Die Ergebnisse entsprechen im Wesentlichen denjenigen, welche analog für den N-terminalen Bereich beobachtet wurden. Bereits ab einer basischen Aminosäure im carboxyterminalen Bereich C (z = 1) wird eine signifikante antimikrobielle Wirkung der Peptide erreicht. Beispielhaft sind hier die MHK-Werte von 32 μM gegenüber E. coli und 8 μM gegenüber S. xylosus des Modellpeptids C4. Durch die Erweiterung des C-terminalen Bereichs auf beispielsweise zwei, drei, vier, oder fünf basische Aminosäuren kann die antimikrobielle Wirkung weiter gesteigert werden. Zum Beispiel weist das Peptid C1 mit fünf basischen Aminosäuren im Bereich C eine MHK von 16 μM beziehungsweise 1 μM auf. Für die Referenzpeptide ohne basische Aminosäure im C-terminalen Bereich konnte keine antimikrobielle Aktivität nachgewiesen werden, sodass das therapeutische Verhältnis lediglich 1 betrug.

**Tabelle 8:** Zusammenfassung der membranzerstörenden Eigenschaften der Peptide C1 bis C8 im Vergleich mit M1. Alle Werte repräsentieren das arithmetische Mittel der Ergebnisse aus vier unabhängigen Testreihen.

| Peptid | Allgemeine Permeabilisierung [μM] | | Äußere Menbran [min] | | | | Innere Menbran [min] | | | |
|---|---|---|---|---|---|---|---|---|---|---|
| | *E. coli* | *S. xylosus* | 4 μM | 8 μM | 16 μM | 32 μM | 4 μM | 8 μM | 16 μM | 32 μM |
| C1 | 25 | 25 | 38 | 30 | 23 | 13 | >90 | >90 | 62 | 45 |
| C2 | 25 | 25 | 40 | 27 | 22 | 16 | >90 | >90 | 64 | 47 |
| M1 | 25 | 25 | 39 | 16 | 17 | 17 | >90 | >90 | 59 | 42 |
| C3 | 50 | 25 | 42 | 30 | 21 | 19 | >90 | >90 | 50 | 45 |
| C4 | 50 | 25 | 41 | 37 | 25 | 12 | >90 | >90 | 53 | 42 |
| C5 | >50 | >50 | >90 | >90 | 26 | 23 | >90 | >90 | >90 | >90 |
| C6 | >50 | >50 | >90 | >90 | >90 | >90 | >90 | >90 | >90 | >90 |
| C7 | >50 | >50 | >90 | >90 | >90 | >90 | >90 | >90 | >90 | >90 |
| C8 | >50 | >50 | >90 | >90 | >90 | >90 | >90 | >90 | >90 | >90 |

Eine derartige Entsprechung findet sich auch in den Ergebnissen der kinetischen Untersuchungen zur Membranpermeabilisierung (Tabelle 8) sowie in der Untersuchung der Sekundärstrukturen, welche für die Peptide C1 und C2 ausgedehnte beta-Stränge zeigen, welche nahezu die gesamte Sequenz des Peptids umfassen (Figur 11). Dagegen weist das Peptid C3 mit nur zwei basischen Aminosäuren am C-Terminus ein verkürztes beta-Strang Motiv in Kombination mit zwei unstrukturierten Regionen an den Termini auf. Das Peptid C4 mit einer basischen Aminosäure am C-Terminus weist noch am N-Terminus einen verkürzten beta-Strang auf, wohingegen der sich daran anschließende Bereich als Random Coil vorliegt.

Beispiel 12

Ausführung des zentralen Bereichs B

Beispielhaft wurde anhand der erfindungsgemäßen Peptide H1 (SEQ ID NO:10), H2 (SEQ ID NO:11) bis H3 (SEQ ID NO:12) sowie der Referenzpeptide H4 (SEQ ID NO:68), H5 (SEQ ID NO:69), H6 (SEQ ID NO:70), H7 (SEQ ID NO:71) bis H8 (SEQ ID NO:72) der Einfluss der Anzahl der hydrophoben beziehungsweise unpolaren Aminosäuren in dem zentralen Bereich B des Peptids systematisch untersucht (Tabelle 9).

**Tabelle 9:** Zusammenfassung der physikalisch-chemischen, antimikrobiellen und zytotoxischen Eigenschaften der Peptide H1 bis H8 im Vergleich zu M1. Alle Werte repräsentieren das arithmetische Mittel der Ergebnisse aus vier unabhängigen Testreihen. [1]Sequenzangabe im Einbuchstabencode vom N-Terminus zum C-Terminus. [2]t.s. kennzeichnet Peptide mit unzureichender Länge für eine akkurate Strukturvorhersage.

| Peptid | Sequenz[1] | Eigenschaften[2] | | MHK [μM] | | Toxizität [μM] | | Therapeutisches Verhältnis | |
|---|---|---|---|---|---|---|---|---|---|
| | | H | μ | *E. coli* | *S. xylosus* | $LD_{50}$ | MTC | *E. coli* | *S. xylosus* |
| H1 | KRKILILILIKRK | 0.486 | 0.054 | 8 | 1 | >64 | >64 | 16 | 128 |
| M1 | KRKILILIKRK | 0.256 | 0.290 | 8 | 2 | >64 | >64 | 16 | 64 |

(fortgesetzt)

| Peptid | Sequenz[1] | Eigenschaften[2] | | MHK [μM] | | Toxizität [μM] | | Therapeutisches Verhältnis | |
|---|---|---|---|---|---|---|---|---|---|
| | | H | μ | E. coli | S. xylosus | LD$_{50}$ | MTC | E. coli | S. xylosus |
| H2 | KRKLILIKRK | 0.102 | 0.209 | >64 | 16 | >64 | >64 | 1 | 8 |
| H3 | KRKILIKRK | -0.008 | 0.045 | >64 | 64 | >64 | >64 | 1 | 2 |
| H4 | KRKLIKRK | t.s. | t.s. | >64 | >64 | >64 | >64 | 1 | 1 |
| H5 | KRKIKRK | t.s. | t.s. | >64 | >64 | >64 | >64 | 1 | 1 |
| H6 | KRKKRK | t.s. | t.s. | >64 | 16 | >64 | >64 | 1 | 8 |
| H7 | KRKGKRK | t.s. | t.s. | >64 | 64 | >64 | >64 | 1 | 2 |
| H8 | KRKGSGKRK | -0.669 | 0.078 | >64 | >64 | >64 | >64 | 1 | 1 |

Bereits ab drei zusammenhängenden hydrophoben und/oder unpolaren Aminosäuren im Bereich B werden die Peptide mit vorteilhaften antimikrobiellen ausgestattet. Beispielsweise weist das Peptid H3 eine MHK von 64 μM gegenüber S. xylosus auf. Durch Hinzunahme weiterer hydrophober und/oder unpolarer Aminosäuren kann eine höhere antimikrobielle Aktivität erreicht werden. Zum Beispiel liegt die MHK des Peptid H2 gegenüber S. xylosus bereits bei 16 μM, während das Peptid H1 mit sieben hydrophoben Aminosäuren mit besonders ausgeprägten antimikrobielle Eigenschaften ausgestattet ist, welche sich in MHK-Werten von 8 μM gegenüber E. coli und 1 μM gegenüber S. xylosus wiederspiegeln. Für die Vergleichspeptide H4 bis H8 mit zwei oder weniger hydrophoben Aminosäuren im zentralen Bereich ließen sich keine den erfindungsgemäßen Peptiden entsprechende antimikrobielle Aktivitäten nachweisen.

Auch die Substitution der hydrophoben und/oder unpolaren Aminosäuren im zentralen Bereich durch neutrale Aminosäuren führte zu keiner Wiederherstellung der antimikrobiellen Aktivität. Eine entsprechende Tendenz wurde auch bei den kinetischen Untersuchungen zur Membranpermeabilisierung im Allgemeinen und der Permeabilisierung der äußeren und inneren Membran im speziellen beobachtet (Tabelle 10).

**Tabelle 10:** Zusammenfassung der membranzerstörenden Eigenschaften der Peptide H1 bis H8 im Vergleich mit M1. Alle Werte repräsentieren das arithmetische Mittel der Ergebnisse aus vier unabhängigen Testreihen.

| Peptid | Allgemeine Permeabilisierung [μM] | | Äußere Membran [min] | | | | Innere Membran [min] | | | |
|---|---|---|---|---|---|---|---|---|---|---|
| | E. coli | S. xylosus | 4 μM | 8 μM | 16 μM | 32 μM | 4 μM | 8 μM | 16 μM | 32 μM |
| H1 | 50 | 5 | 26 | 20 | 19 | 17 | >90 | 37 | 35 | 33 |
| M1 | 25 | 25 | 39 | 16 | 17 | 17 | >90 | >90 | 59 | 42 |
| H2 | >50 | 25 | >90 | >90 | >90 | >90 | >90 | >90 | >90 | >90 |
| H3 | >50 | 50 | >90 | >90 | >90 | >90 | >90 | >90 | >90 | >90 |
| H4 | >50 | >50 | >90 | >90 | >90 | >90 | >90 | >90 | >90 | >90 |
| H5 | >50 | >50 | >90 | >90 | >90 | >90 | >90 | >90 | >90 | >90 |
| H6 | >50 | >50 | >90 | >90 | >90 | >90 | >90 | >90 | >90 | >90 |
| H7 | >50 | >50 | >90 | >90 | >90 | >90 | >90 | >90 | >90 | >90 |
| H8 | >50 | >50 | >90 | >90 | >90 | >90 | >90 | >90 | >90 | >90 |

Die Sekundärstruktur des Peptids H1 ist durch jeweils einen beta-Strang im C- und N-terminalen Bereich und einem dazwischenliegenden unstrukturierten Bereich gekennzeichnet, während das Peptid H2 über nahezu die gesamte Sequenz einen einzigen beta-Strang ausbildet (Figur 12). Das Peptid H3 weist einen ausgedehnten beta-Strang im zentralen Bereich auf, welcher lediglich von zwei kurzen unstrukturierten Bereichen flankiert wird. Im Gegensatz hierzu liegt das Peptid H8, welches zwar jeweils drei basische Aminosäuren im C-terminalen Bereich und N-terminalen Bereich, jedoch im zentralen Bereich lediglich neutrale Aminosäuren aufweist, vollständig als Random Coil vor.

Beispiel 13

Ausführung hinsichtlich der Anordnung von hydrophoben und/oder unpolaren Aminosäuren und basischen Aminosäuren.

Im nächsten Schritt wurde die Rolle der Anordnung von hydrophoben und basischen Aminosäuren innerhalb der Peptidsequenz untersucht. Die Ergebnisse sind in Tabelle 11 beispielhaft für das erfindungsgemäße Peptid A1 (SEQ ID NO:13) sowie die Referenzpeptide A2 (SEQ ID NO:14), A3 (SEQ ID NO:73), A4 (SEQ ID NO:74) bis A5 (SEQ ID NO: 75) gezeigt. Bei diesen Peptiden wurden die hydrophoben Aminosäuren schrittweise vom zentralen Bereich der Sequenz in die amino- beziehungsweise carboxyterminalen Bereiche und die basischen Aminosäuren von den terminalen Bereichen entsprechend in den zentralen Bereich verschoben. Auf diese Weise resultieren Peptide gleicher Länge und Hydrophobizität, aber mit unterschiedlichen hydrophoben Momenten und Primärsequenzen.

**Tabelle 11:** Zusammenfassung der physikalisch-chemischen, antimikrobiellen und zytotoxischen Eigenschaften der Peptide A1 bis A5. Alle Werte repräsentieren das arithmetische Mittel der Ergebnisse aus vier unabhängigen Testreihen. [1]Sequenzangabe im Einbuchstabencode vom N-Terminus zum C-Terminus.

| Peptid | Sequenz[1] | Eigenschaften | | MHK [μM] | | Toxizität [μM] | | Therapeutisches Verhältnis | |
|---|---|---|---|---|---|---|---|---|---|
| | | H | μ | E. coli | S. xylosus | LD$_{50}$ | MTC | E. coli | S. xylosus |
| A1 | RKRKLILILIKRKR | 0.179 | 0.191 | 8 | 0.5 | >64 | >64 | 16 | 256 |
| A2 | KRKLILKRILIKRK | 0.179 | 0.054 | 16 | 8 | >64 | >64 | 8 | 16 |
| A3 | RKLILKRKRILIKR | 0.179 | 0.283 | 16 | 8 | >64 | >64 | 8 | 16 |
| A4 | KLILKRKRKRILIK | 0.179 | 0.034 | 32 | 16 | >64 | >64 | 4 | 8 |
| A5 | LILKRKRKRKRILI | 0.179 | 0.221 | >64 | >64 | >64 | >64 | 1 | 1 |

Es ist ersichtlich, dass auch die Unterbrechung der Abfolge hydrophober beziehungsweise unpolarer Aminosäuren im zentralen Bereich B durch eine oder mehrere andere Aminosäuren, beispielsweise basische Aminosäuren, die Bereitstellung antimikrobiell wirksamer Peptide gewährleistet, solange B mindestens 80% hydrophobe/unpolare Aminosäuren und eine direkte Aufeinanderfolge von mindestens drei hydrophoben/unpolaren Aminosäuren aufweist. Die Durchbrechung des erfindungsgemäßen Strukturmotivs durch Anordnung von lediglich 60% hydrophoben und/oder unpolaren Aminosäuren im Bereich B, z.B. Peptid A3, beziehungsweise 40% hydrophobe und/oder unpolare Aminosäuren im Bereich B, z.B. Peptid A4, resultiert in einer Verschlechterung der MHK-Werte gegenüber E. coli und S. xylosus. Der vollständige Tausch der hydrophoben und basischen Aminosäuren im Peptid A5 führt zu einem Komplettverlust der antimikrobiellen Eigenschaften. Entsprechende Trends spiegeln sich auch in den kinetische Eigenschaften hinsichtlich der Permeabilisierung der äußeren und inneren Bakterienmembran der erfindungsgemäßen Peptide wieder (Tabelle 12).

**Tabelle 12:** Zusammenfassung der membranzerstörenden Eigenschaften der Peptide A1 bis A5. Alle Werte repräsentieren das arithmetische Mittel der Ergebnisse aus vier unabhängigen Testreihen.

| Peptid | Permeabilisierung [μM] | | Äußere Membran [min] | | | | Innere Membran [min] | | | |
|---|---|---|---|---|---|---|---|---|---|---|
| | *E. coli* | *S. xylosus* | 4 μM | 8 μM | 16 μM | 32 μM | 4 μM | 8 μM | 16 μM | 32 μM |
| A1 | 25 | 5 | 26 | 21 | 17 | 16 | >90 | 42 | 40 | 35 |
| A2 | >50 | 25 | >90 | >90 | 21 | 16 | >90 | >90 | 60 | 80 |
| A3 | >50 | 50 | >90 | >90 | >90 | 17 | >90 | >90 | >90 | 72 |
| A4 | >50 | >50 | >90 | >90 | >90 | >90 | >90 | >90 | >90 | >90 |
| A5 | >50 | >50 | >90 | >90 | >90 | >90 | >90 | >90 | >90 | >90 |

Die Sekundärstruktur des Peptids A1 ist weitestgehend durch beta-Stränge charakterisiert, während die Durchbrechung des hydrophoben zentralen Bereichs mit zwei basischen Aminosäuren beim Peptid A2 zu einer Strukturänderung hin zu einem alphahelikalen Motiv führt (Figur 13).

Beispiel 14

Ausführung der Aminosäurezusammensetzung

Für die Untersuchung des Einflusses einzelner Aminosäuren auf die antimikrobiellen und zytotoxischen Eigenschaften der antimikrobiellen Peptide wurde ein modellhaftes Peptidmotiv mit jeweils drei basischen Aminosäuren in den Bereichen A und C sowie sechs hydrophoben/unpolaren Aminosäuren im zentralen Bereich B verwendet. Auf der Grundlage dieses Motivs wurden Lysin und Arginin als beispielhafte basische Aminosäuren sowie Isoleucin, Leucin, Phenylalanin und Valin als beispielhafte hydrophobe/unpolare Aminosäuren kombinatorisch zusammengestellt und die antimikrobiellen Eigenschaften der entsprechenden erfindungsgemäßen Peptide S1 (SEQ ID NO:15), S2 (SEQ ID NO:16), S3 (SEQ ID NO:17), S4 (SEQ ID NO:18), S5 (SEQ ID NO:19), S6 (SEQ ID NO:20), S7 (SEQ ID NO:21) und S8 (SEQ ID NO:22) untersucht (Tabelle 13).

**Tabelle 13:** Zusammenfassung der physikalisch-chemischen, antimikrobiellen und zytotoxischen Eigenschaften der Peptide S1 bis S8. Alle Werte repräsentieren das arithmetische Mittel der Ergebnisse aus vier unabhängigen Testreihen. [1]Sequenzangabe im Einbuchstabencode vom N-Terminus zum C-Terminus. [2]Werte in Klammern bedeuten, dass zwar eine MTC aber keine $LD_{50}$ festgestellt wurde.

| Peptid | Sequenz[1] | Eigenschaften | | MHK [μM] | | Toxizität [μM] | | Therapeutisches Verhältnis[2] | |
|---|---|---|---|---|---|---|---|---|---|
| | | H | μ | E. coli | *S. xylosus* | $LD_{50}$ | MTC | *E. coli* | *S. xylosus* |
| S1 | RRRIIIIRRR | 0.267 | 0.293 | 8 | 2 | >64 | 64 | (16) | (64) |
| S2 | KKKIIIIKKK | 0.278 | 0.291 | 8 | 0.5 | >64 | >64 | 16 | 256 |
| S3 | RRRLLLLLRRR | 0.222 | 0.281 | 8 | 1 | >64 | 64 | (16) | (128) |

(fortgesetzt)

| Peptid | Sequenz[1] | Eigenschaften | | MHK [$\mu$M] | | Toxizität [$\mu$M] | | Therapeutisches Verhältnis[2] | |
|---|---|---|---|---|---|---|---|---|---|
| | | H | $\mu$ | E. coli | S. xylosus | LD$_{50}$ | MTC | E. coli | S. xylosus |
| S4 | KKKLLLLLKKK | 0.233 | 0.280 | 16 | 8 | >64 | >64 | 4 | 8 |
| S5 | RRRVVVVVRRR | 0.004 | 0.228 | 32 | 2 | >64 | >64 | 4 | 64 |
| S6 | KKKVVVVVKKK | 0.015 | 0.226 | 64 | 8 | >64 | >64 | 2 | 16 |
| S7 | RRRFFFFFRRR | 0.263 | 0.291 | 4 | 0.5 | >64 | 32 | (32) | (256) |
| S8 | KKKFFFFFKKK | 0.274 | 0.290 | 16 | 4 | >64 | >64 | 8 | 32 |

Grundsätzlich wurde mit allen Peptiden S1 bis S8 eine vorteilhafte antimikrobielle Wirkung erreicht, wobei die MHK-Werte zwischen 4 $\mu$M und 64 $\mu$M für E. coli und 0,5 $\mu$M bis 8 $\mu$M für S. xylosus lagen. Hinsichtlich der basischen Aminosäuren führte die Verwendung von Arginin gegenüber Lysin zu einer stärkeren antimikrobiellen Wirkung, was sich in den MHK-Werten in Tabelle 13 und den Ergebnissen der kinetischen Untersuchung in Tabelle 14 wiederspiegelt. Gleichzeitig weisen die Peptide S1, S3 und S7 mit Arginin als basische Aminosäure jedoch auch wachstumsinhibierende Eigenschaften gegenüber der eukaryotischen Zelllinie U937 auf. Hier zeigt sich wiederum ein Vorteil der Lysin enthaltenen Peptide S2, S4, S6 und S8, welche keine entsprechenden zytotoxischen Eigenschaften aufweisen.

**Tabelle 14:** Zusammenfassung der membranzerstörenden Eigenschaften der Peptide S1 bis S8. Alle Werte repräsentieren das arithmetische Mittel der Ergebnisse aus vier unabhängigen Testreihen.

| Peptid | Allgemeine Permeabilisierung [$\mu$M] | | Äußere Membran [min] | | | | Innere Membran [min] | | | |
|---|---|---|---|---|---|---|---|---|---|---|
| | E. coli | S. xylosus | 4 $\mu$M | 8 $\mu$M | 16 $\mu$M | 32 $\mu$M | 4 $\mu$M | 8 $\mu$M | 16 $\mu$M | 32 $\mu$M |
| S1 | 25 | 25 | 25 | 24 | 22 | 19 | >90 | 63 | 61 | 56 |
| S2 | 25 | 5 | 26 | 23 | 21 | 21 | >90 | 62 | 58 | 51 |
| S3 | 50 | 25 | >90 | 32 | 22 | 15 | >90 | >90 | >90 | 83 |
| S4 | 50 | 50 | >90 | >90 | >90 | 24 | >90 | >90 | >90 | 91 |
| S5 | 50 | 25 | >90 | >90 | >90 | 19 | >90 | >90 | >90 | >90 |
| S6 | >50 | >50 | >90 | >90 | >90 | 31 | >90 | >90 | >90 | >90 |
| S7 | 25 | 25 | >90 | 14 | 13 | 9 | >90 | 39 | 37 | 34 |
| S8 | 50 | 50 | >90 | >90 | >90 | 20 | >90 | >90 | >90 | 64 |

Beispielsweise können daher solche antimikrobiellen Peptide, welche mit vermehrtem Anteil an Lysin als basische Aminosäure in den Bereichen A und C ausgeführt sind, aufgrund ihrer hohen Selektivität gegenüber bakteriellen Membranen besonders vorteilhaft im medizinisch-therapeutischen Bereich eingesetzt werden, da zum Beispiel humane Zellen nicht beschädigt werden. Aufgrund ihrer starken antimikrobiellen Eigenschaften sind Peptide mit vermehrtem Anteil an Arginin als basische Aminosäure in den Bereichen A und C besonders für ex vivo Anwendungen geeignet, beispielsweise als Bestandteil antimikrobieller Zusammensetzungen und Beschichtungen für das Antifouling.

In Bezug auf den zentralen Bereich B werden besonders vorteilhafte antimikrobielle Eigenschaften mit den Aminosäuren Isoleucin, Leucin und Phenylalanin erreicht.

Beispiel 15

Ausführung mit sauren Aminosäuren

Das erfindungsgemäße Strukturmotiv wurde weiterhin hinsichtlich der Ausgestaltungsmöglichkeiten mit sauren Aminosäuren, das heißt negativ geladenen Resten, analysiert. In Tabelle 14 sind repräsentative Ergebnisse für eine Grundstruktur mit fünf basischen Aminosäuren jeweils im aminoterminalen Bereich A und carboxyterminalen Bereich C sowie fünf hydrophoben/unpolaren Aminosäuren im zentralen Bereich B gezeigt. Von dieser Grundstruktur wurden exemplarische Variationen durchgeführt, indem basische Aminosäuren im carboxyterminalen Bereich schrittweise durch saure Aminosäuren, beispielsweise Asparaginsäure, ausgetauscht wurden. Auf diese Weise wurden die erfindungsgemäßen Peptide D4 (SEQ ID NO:23), D5 (SEQ ID NO:24), D6 (SEQ ID NO:25) und D7 (SEQ ID NO:26) sowie die Referenzpeptide D1 (SEQ ID NO:76), D2 (SEQ ID NO:77) und D3 (SEQ ID NO:78) sowie D8 (SEQ ID NO:79) generiert.

**Tabelle 15:** Zusammenfassung der physikalisch-chemischen, antimikrobiellen und zytotoxischen Eigenschaften der Peptide D1 bis D8. Alle Werte repräsentieren das arithmetische Mittel der Ergebnisse aus vier unabhängigen Testreihen. [1]Sequenzangabe im Einbuchstabencode vom N-Terminus zum C-Terminus.

| Peptid | Sequenz[1] | Eigenschaften | | MHK [μM] | | Toxizität [μM] | | Therapeutisches Verhältnis | |
|---|---|---|---|---|---|---|---|---|---|
| | | H | μ | E. coli | S. xylosus | $LD_{50}$ | MTC | E. coli | S. xylosus |
| D1 | KKKKKIIIIIDDDDD | 0.013 | 0.258 | >64 | >64 | >64 | >64 | 1 | 1 |
| D2 | KKKKKIIIIIKDDDD | -0.001 | 0.265 | >64 | >64 | >64 | >64 | 1 | 1 |
| D3 | KKKKKIIIIIKKKDD | -0.031 | 0.265 | >64 | 32 | >64 | >64 | 1 | 4 |
| D4 | KKKKKIIIIIKKKKD | -0.045 | 0.258 | 32 | 16 | >64 | >64 | 4 | 8 |
| D5 | KKKKKIIIIIKKKKK | -0.060 | 0.271 | 16 | 2 | >64 | >64 | 8 | 64 |
| D6 | KKKKKIIIIIDKKKK | -0.045 | 0.264 | 32 | 16 | >64 | >64 | 4 | 8 |
| D7 | KKKKKIIIIIDDDKK | -0.016 | 0.264 | >64 | >64 | >64 | >64 | 1 | 1 |
| D8 | KKKKKIIIIIDDDDK | -0.001 | 0.271 | >64 | >64 | >64 | >64 | 1 | 1 |

Das Peptid D5 mit der oben genannten Grundstruktur weist eine hohe antimikrobielle Aktivität und gleichzeitig keine cytotoxischen Eigenschaften auf. Die MHK-Werte liegen bei 16 μM für E. coli und 2 μM für S. xylosus. Der Austausch von einer basischen Aminosäure mit einer sauren Aminosäure im Übergang des zentralen Bereichs B in den carboxyterminalen Bereich C beziehungsweise am Carboxyterminus von C resultiert in einer vorteilhaften, wenn auch leicht verringerten antimikrobiellen Aktivität mit MHK-Werten von 32 μM beziehungsweise 16 μM (vergleiche Peptide D6 und D4). Der weitere Austausch basischer gegen saure Aminosäuren führt zu einer Verringerung der antimikrobiellen Wirksamkeit, so dass bei einem Anteil von unter 60% basischer Aminosäuren im carboxyterminalen Bereich C praktisch keine antimikrobielle Aktivität mehr nachgewiesen werden kann.

**Tabelle 16:** Zusammenfassung der membranzerstörenden Eigenschaften der Peptide D1 bis D8. Alle Werte repräsentieren das arithmetische Mittel der Ergebnisse aus vier unabhängigen Testreihen.

| Peptid | Allgemeine Permeabilisierung [μM] | | Äußere Membran [min] | | | | Innere Membran [min] | | | |
|---|---|---|---|---|---|---|---|---|---|---|
| | *E. coli* | *S. xylosus* | 4 μM | 8 μM | 16 μM | 32 μM | 4 μM | 8 μM | 16 μM | 32 μM |
| D1 | >50 | >50 | >90 | >90 | >90 | >90 | >90 | >90 | >90 | >90 |
| D2 | >50 | >50 | >90 | >90 | >90 | >90 | >90 | >90 | >90 | >90 |
| D3 | >50 | >50 | >90 | >90 | >90 | >90 | >90 | >90 | >90 | >90 |
| D4 | 50 | 25 | >90 | >90 | >90 | >90 | >90 | >90 | >90 | >90 |
| D5 | 25 | 5 | 32 | 29 | 28 | 26 | >90 | 68 | 66 | 59 |
| D6 | >50 | 25 | >90 | >90 | >90 | >90 | >90 | >90 | >90 | >90 |
| D7 | >50 | >50 | >90 | >90 | >90 | >90 | >90 | >90 | >90 | >90 |
| D8 | >50 | >50 | >90 | >90 | >90 | >90 | >90 | >90 | >90 | >90 |

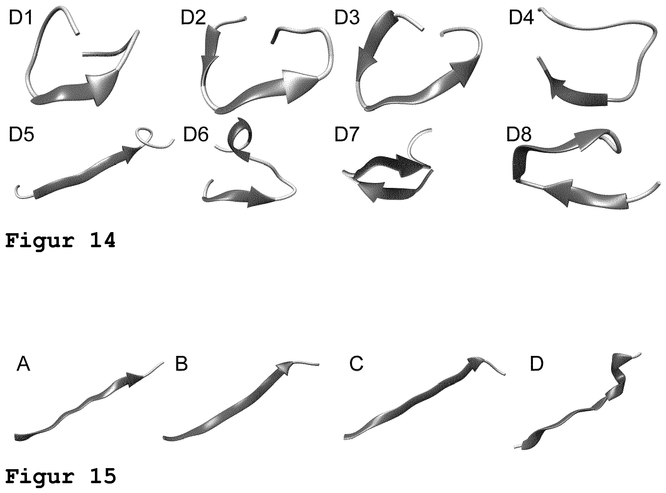

Aus Figur 14 kann wiederum entnommen werden, dass die Abnahme der antimikrobiellen Aktivität gegenüber dem Peptid D5 strukturell mit einer Verringerung beziehungsweise Durchbrechung des gestreckten beta-Strangs korreliert.

Beispiel 16

Behandlung bakteriell kontaminierter Zellkultur

Das medizinisch-therapeutische Potential der erfindungsgemäßen Peptide wurde mit Hilfe von Mischkulturexperimenten wie in Beispiel 7 beschrieben untersucht. Beispielhaft sind hier in Tabelle 17 und Tabelle 18 die Ergebnisse der Untersuchung des erfindungsgemäßen Peptids O1 (SEQ ID NO:27) mit einem zentralen Bereich B aus sechs hydrophoben/unpolaren Aminosäuren und den flankierenden Bereichen A und C mit jeweils drei basischen Aminosäuren gezeigt. Des Weiteren wurde das Peptid O1' untersucht, welches dieselbe Sequenz besitzt, wobei aber der Carboxyterminus anstatt als freie Säure (-COOH) als Amid (-CONH$_2$) ausgebildet wurde.

**Tabelle 17:** Zusammenfassung der physikalisch-chemischen, antimikrobiellen und zytotoxischen Eigenschaften des Peptids O1 und die antimikrobiellen Eigenschaften des Peptids O1'. Alle Werte repräsentieren das arithmetische Mittel der Ergebnisse aus vier unabhängigen Testreihen. [1]Sequenzangabe im Einbuchstabencode vom N-Terminus zum C-Terminus.

| Peptid | Sequenz[1] | Eigenschaften | | MHK [μM] | | Toxizität [μM] | | Therapeutisches Verhältnis | |
|---|---|---|---|---|---|---|---|---|---|
| | | H | μ | *E. coli* | *S. xylosus* | LD$_{50}$ | MTC | *E. coli* | *S. xylosus* |
| O1 | KKKIIIIIKKK | 0.405 | 0.170 | 8 | 0.5 | >64 | >64 | 16 | 256 |
| O1' | KKKIIIIIKKK -CONH2 | n. best. | n. best. | 4 | 0.25 | n. best. | n. best. | n. best. | n. best. |

Es wurde für das Peptid O1 eine hohe antimikrobielle Aktivität mit MHK-Werten zwischen 8 μM und 0,5 μM

festgestellt. Überraschenderweise konnte die antimikrobielle Aktivität des Peptids O1 durch die Amidierung des Carboxyterminus, repräsentiert durch O1', nochmals verdoppelt werden, was sich in einer weiteren Senkung der MHK-Werte auf 4 μM beziehungsweise 0,25 μM äußerte.

Das Peptid O1 zeigte in Bezug auf E. coli und S. xylosus eine ausgeprägte Membranzerstörung bei Peptidkonzentrationen von 25 μM beziehungsweise 5 μM. In den kinetischen Untersuchungen konnte eine Permeabilisierung der äußeren und inneren Membran von E. coli ML-35p bereits ab einer Konzentration von 4 μM festgestellt werden.

**Tabelle 18:** Zusammenfassung der membranzerstörenden Eigenschaften des Peptids O1. Alle Werte repräsentieren das arithmetische Mittel der Ergebnisse aus vier unabhängigen Testreihen.

| Peptid | Allgemeine Permeabilisierung [μM] | | Äußere Membran [min] | | | | Innere Membran [min] | | | |
|---|---|---|---|---|---|---|---|---|---|---|
| | *E. coli* | *S. xylosus* | 4 μM | 8 μM | 16 μM | 32 μM | 4 μM | 8 μM | 16 μM | 32 μM |
| O1 | 25 | 5 | 23 | 21 | 19 | 17 | 41 | 39 | 39 | 39 |

Gleichzeitig konnten keine zytotoxischen Eigenschaften gegenüber der eukaryotischen Zelllinie U937 festgestellt werden, sodass besonders vorteilhafte therapeutische Verhältnisse von 16 beziehungsweise 256 resultierten.

Die Sekundärstruktur des Peptids bestätigt die ausgeprägte beta-Strang Struktur der erfindungsgemäßen Peptide im Zusammenhang mit der antimikrobiellen Wirksamkeit (Figur 15).

Diese hochselektive Wirkung gegenüber bakteriellen Zellmembranen wird durch die Mischkulturexperimente bestätigt (Figur 7). In dem Säulendiagramm sind beispielhaft die Verhältnisse von lebenden und toten Bakterienzellen (S. xylosus) und eukaryotischen U937 Zellen in der Mischkultur für die Negativkontrolle (NC) sowie nach Zugabe von 25 und 50 μM Melittin beziehungsweise Peptid O1 dargestellt. Die Kontrollexperimente (NC) zeigen im Wesentlichen nur lebende Bakterien- und eukaryotische U937 Zellen. Das natürliche antimikrobielle Referenzpeptid Melittin führt in beiden getesteten Konzentrationen zwar zur umfassenden Abtötung von Bakterienzellen, jedoch zerstört das Melittin gleichzeitig auch alle eukaryotischen U937 Zellen. Im Gegensatz hierzu zeigt sich, dass ein erfindungsgemäßes Peptid bereits ab 25 μM Konzentration zu einer nahezu vollständigen Abtötung der S. xylosus Zellen führt, wohingegen die eukaryotischen U937 Zellen von der Behandlung mit dem Peptid unbeeinflusst bleiben. Somit konnte gezeigt werden, dass sich die antimikrobielle Eigenschaft der erfindungsgemäßen Peptide hochselektiv gegenüber Bakterienzellen auswirkt und eukaryotische Zellen nicht beschädigt. Hieraus ergeben sich besondere Vorteile für die medizinisch-therapeutische Verwendung der erfindungsgemäßen Peptide. Beispielsweise können im Gegensatz zu herkömmlichen antimikrobiellen Peptiden zytotoxische Nebenwirkungen bei einer Antibiose wirksam vermieden werden, was zu einer verbesserten Verträglichkeit und höheren Patientensicherheit beiträgt.

Die Erfindung ist nicht durch die Beschreibung anhand der Ausführungsbeispiele beschränkt. Vielmehr umfasst die Erfindung jedes neue Merkmal sowie jede Kombination von Merkmalen, was insbesondere jede Kombination von Merkmalen in den Patentansprüchen beinhaltet, auch wenn dieses Merkmal oder diese Kombination selbst nicht explizit in den Patentansprüchen oder Ausführungsbeispielen angegeben ist.

Literatur

(Ahmad et al. 2009) Ahmad, A., Azmi, S., Srivastava, R. M., Srivastava, S., Pandey, B. K., Saxena, R., Bajpai, V. K., and Ghosh, J. K. (2009) Design of nontoxic analogues of cathelicidin-derived bovine antimicrobial peptide BMAP-27: the role of leucine as well as phenylalanine zipper sequences in determining its toxicity. Biochemistry 48, 10905-17.

(Bell and Gouyon 2003) Bell, G., and Gouyon, P. H. (2003) Arming the enemy: the evolution of resistance to selfproteins. Microbiology 149, 1367-75.

(Brogden et al. 2003) Brogden, K. A., Ackermann, M., McCray, P. B., Jr., and Tack, B. F. (2003) Antimicrobial peptides in animals and their role in host defences. Int J Antimicrob Agents 22, 465-78.

(Brogden 2005) Brogden, K. A. (2005) Antimicrobial peptides: pore formers or metabolic inhibitors in bacteria? Nat Rev Microbiol 3, 238-50.

(Brotz et al. 1998) Brotz, H., Bierbaum, G., Leopold, K., Reynolds, P. E., and Sahl, H. G. (1998) The lantibiotic mersacidin inhibits peptidoglycan synthesis by targeting lipid II. Antimicrob Agents Chemother 42, 154-60.

(Clinical and Laboratory Standards Institute 2006) Clinical and Laboratory Standards Institute. (2006) Performance standards for antimicrobial susceptibility testing; sixteenth informational supplement. CLSI document M100-S16CLSI, Wayne, PA.

(Conlon et al. 2003) Conlon, J. M., Sonnevend, A., Patel, M., Camasamudram, V., Nowotny, N., Zilahi, E., Iwamuro, S., Nielsen, P. F., and Pál, T. (2003) A melittin-related peptide from the skin of the Japanese frog, Rana tagoi, with antimicrobial and cytolytic properties. Biochemical and Biophysical Research Communications 306, 496-500.

(Dathe 1997) Dathe, M., Wieprecht, T., Nikolenko, H., Handel, L., Maloy, W. L., MacDonald, D. L., Beyermann, M., and Bienert, M. (1997) Hydrophobicity, hydrophobic moment and angle subtended by charged residues modulate antibacterial and haemolytic activity of amphipathic helical peptides. FEBS Lett 403, 208-12.

(Diamond 2001) Diamond, G. (2001) Natures antibiotics: the potential of antimicrobial peptides as new drugs. Biologist (London) 48, 209-12.

(European Committee for Antimicrobial Susceptability Testing of the European Society of Clinical Microbiology and Infectious Diseases 2003) European Committee for Antimicrobial Susceptibility Testing of the European Society of Clinical, M., and Infectious, D. (2003) Determination of minimum inhibitory concentrations (MICs) of antibacterial agents by broth dilution. Clinical Microbiology and Infection 9, ix-xv.

(Gautier et al. 2008) Gautier, R., Douguet, D., Antonny, B., and Drin, G. (2008) HELIQUEST: a web server to screen sequences with specific alpha-helical properties. Bioinformatics 24, 2101-2.

(Gennaro and Zanetti 2000) Gennaro, R., and Zanetti, M. (2000) Structural features and biological activities of the cathelicidin-derived antimicrobial peptides. Biopolymers 55, 31-49.

(Ginsburg and Koren 2008) Ginsburg, I., and Koren, E. (2008) Are cationic antimicrobial peptides also 'double-edged swords'? Expert Rev Anti Infect Ther 6, 453-62.

(Giuliani et al. 2007) Giuliani, A., Pirri, G., and Nicoletto, S. (2007) Antimicrobial peptides: an overview of a promising class of therapeutics. Central European Journal of Biology 2, 1-33.

(Gordon et al. 2005) Gordon Yj Fau - Romanowski, E. G., Romanowski Eg Fau - McDermott, A. M., and McDermott, A. M. (2005) A review of antimicrobial peptides and their therapeutic potential as anti-infective drugs. Curr Eye Res 30, 505-515.

(Hilpert et al. 2010) Hilpert, K., McLeod, B., Yu, J., Elliott, M. R., Rautenbach, M., Ruden, S., Burck, J., Muhle-Goll, C., Ulrich, A. S., Keller, S., and Hancock, R. E. (2010) Short cationic antimicrobial peptides interact with ATP. Antimicrob Agents Chemother 54, 4480-3.

(Jin et al. 2005) Jin, Y., Hammer, J., Pate, M., Zhang, Y., Zhu, F., Zmuda, E., and Blazyk, J. (2005) Antimicrobial activities and structures of two linear cationic peptide families with various amphipathic beta-sheet and alpha-helical potentials. Antimicrob Agents Chemother 49, 4957-64.

(Kaur et al. 2007) Kaur, H., Garg, A., and Raghava, G. P. (2007) PEPstr: a de novo method for tertiary structure prediction of small bioactive peptides. Protein Pept Lett 14, 626-31.

(Lehrer and Ganz 2002) Lehrer, R. I., and Ganz, T. (2002) Defensins of vertebrate animals. Curr Opin Immunol 14, 96-102.

(Lottspeich and Engels 2006) Lottspeich, F., Engels, J. W. (Hrsg.) (2006) Bioanalytik. Spektrum Akademischer Verlag.

(Marr et al. 2006) Marr, A. K., Gooderham, W. J., and Hancock, R. E. W. (2006) Antibacterial peptides for therapeutic use: obstacles and realistic outlook. Current Opinion in Pharmacology 6, 468-472.

(Maupetit et al. 2009) Maupetit, J., Derreumaux, P., and Tuffery, P. (2009) PEP-FOLD: an online resource for de novo peptide structure prediction. Nucleic Acids Res 37, W498-503.

(Maupetit et al. 2010) Maupetit, J., Derreumaux, P., and Tuffery, P. (2010) A fast method for large-scale de novo peptide and miniprotein structure prediction. J Comput Chem 31, 726-38.

(Ong et al. 2013) Ong, Z. Y., Gao, S. J., and Yang, Y. Y. (2013) Short Synthetic β-Sheet Forming Peptide Amphiphiles as Broad Spectrum Antimicrobials with Antibiofilm and Endotoxin Neutralizing Capabilities. Advanced Functional Materials 23, 3682-3692.

(Otvos et al. 2000) Otvos, L., Jr., O, I., Rogers, M. E., Consolvo, P. J., Condie, B. A., Lovas, S., Bulet, P., and Blaszczyk-Thurin, M. (2000) Interaction between heat shock proteins and antimicrobial peptides. Biochemistry 39, 14150-9.

(Oyston et al. 2009) Oyston, P. C., Fox, M. A., Richards, S. J., and Clark, G. C. (2009) Novel peptide therapeutics for treatment of infections. J Med Microbiol 58, 977-87.

(Pag et al. 2004) Pug, U., Oedenkoven, M., Papo, N., Oren, Z., Shai, Y., and Sahl, H.-G. (2004) In vitro activity and mode of action of diastereomeric antimicrobial peptides against bacterial clinical isolates. Journal of Antimicrobial Chemotherapy 53, 230-239.

(Pettersen et al. 2004) Pettersen, E. F., Goddard, T. D., Huang, C. C., Couch, G. S., Greenblatt, D. M., Meng, E. C., and Ferrin, T. E. (2004) UCSF Chimera-A visualization system for exploratory research and analysis. Journal of Computational Chemistry 25, 1605-1612

(Rausch et al. 2005) Rausch, J. M., Marks, J. R., and Wimley, W. C. (2005) Rational combinatorial design of pore-forming beta-sheet peptides. Proc Natl Acad Sci U S A 102, 10511-5.

(Rausch et al. 2007) Rausch, J. M., Marks, J. R., Rathinakumar, R., and Wimley, W. C. (2007) Beta-sheet pore-forming peptides selected from a rational combinatorial library: mechanism of pore formation in lipid vesicles and activity in biological membranes. Biochemistry 46, 12124-39.

(Richter 2003) Richter, G. (2003) Praktische Biochemie. Georg Thieme Verlag.

(Shai 1999) Shai, Y. (1999) Mechanism of the binding, insertion and destabilization of phospholipid bilayer membranes by alpha-helical antimicrobial and cell nonselective membrane-lytic peptides. Biochim Biophys Acta 1462, 55-70.

(Stryer 1999) Stryer, L. (1999) Biochemie. Spektrum Akademischer Verlag Heidelberg.

(Subbalakshmi and Sitaram 1998) Subbalakshmi, C., and Sitaram, N. (1998) Mechanism of antimicrobial action of indolicidin. FEMS Microbiol Lett 160, 91-6.

(Thevenet et al. 2012) Thevenet, P., Shen, Y., Maupetit, J., Guyon, F., Derreumaux, P., and Tuffery, P. (2012) PEP-FOLD: an updated de novo structure prediction server for both linear and disulfide bonded cyclic peptides. Nucleic Acids Res 40, W288-93.

(Thompson et al. 1994) Thompson, J.D., Higgins, D.G., Gibson, T.J. (1994) CLUSTAL W: improving the sensitivity of progressive multiple sequence alignment through sequence weighting, position-specific gap penalties and weight matrix choice. Nucleic Acid Research 22, 4673-4680

(Tossi et al. 2000) Tossi, A., Sandri, L., and Giangaspero, A. (2000) Amphipathic, alpha-helical antimicrobial peptides. Biopolymers 55, 4-30.

(Wiegand et al. 2008) Wiegand, I., Hilpert, K., and Hancock, R. E. (2008) Agar and broth dilution methods to determine the minimal inhibitory concentration (MIC) of antimicrobial substances. Nat Protoc 3, 163-75.

(Wiradharma 2013) Wiradharma, N., Sng, M. Y. S., Khan, M., Ong, Z. Y., and Yang, Y. Y. (2013) Rationally Designed alpha-Helical Broad-Spectrum Antimicrobial Peptides with Idealized Facial Amphiphilicity. Macromolecular Rapid Communications 34, 74-80.

(Zaiou 2007) Zaiou, M. (2007) Multifunctional antimicrobial peptides: therapeutic targets in several human diseases. J Mol Med (Berl) 85, 317-29.

**Patentansprüche**

1. Ein Peptid mit einem aminoterminalen Bereich A, einem carboxyterminalen Bereich C und einem dazwischenliegenden Bereich B der allgemeinen Formel

$$A_xB_yC_z,$$

mit x, z $\geq$ 1, wobei x $\geq$ 3 und/oder z $\geq$ 3, y $\geq$ 3 und x + y + z $\leq$ 50,
wobei A und C jeweils unabhängig voneinander mindestens 65%, 75% oder 80% basische Aminosäuren aufweisen,
wobei B mindestens 80% hydrophobe und/oder unpolare Aminosäuren ausgewählt aus der Gruppe bestehend aus Val, Ile, Phe und beliebigen Kombinationen von Val, Leu, Ile und Phe aufweist und eine direkte Aufeinanderfolge von mindestens drei, vier oder fünf hydrophoben Aminosäuren umfasst.

2. Das Peptid nach Anspruch 1 mit y $\geq$ 4 oder y $\geq$ 5.

3. Das Peptid nach einem der Ansprüche 1 oder 2, wobei die aminoterminale Aminosäure in A und/oder die carboxyterminale Aminosäure in C eine basische Aminosäure ist.

4. Das Peptid nach einem der Ansprüche 1 bis 3, wobei die basischen Aminosäuren unabhängig voneinander ausgewählt sind aus der Gruppe bestehend aus Lys, Arg und His oder aus Lys und Arg.

5. Das Peptid nach einem der Ansprüche 1 bis 4, wobei A und/oder C ausschließlich basische Aminosäuren umfasst und/oder wobei B ausschließlich hydrophobe und/oder unpolare Aminosäuren umfasst.

6. Das Peptid nach einem der Ansprüche 1 bis 5 mit y $\leq$ 20, y $\leq$ 15 oder y $\leq$ 10 und/oder mit x + y + z $\leq$ 30 oder x + y + z $\leq$ 20.

7. Das Peptid nach einem der Ansprüche 1 bis 6, wobei in einem wässrigen Milieu das Peptid zumindest teilweise als beta-Strang ausgebildet ist, vorzugsweise mindestens 50%, 65%, 75% oder 80% der Aminosäuren des Peptids in einem einzigen beta-Strang umfasst sind.

**8.** Das Peptid nach einem der Ansprüche 1 bis 7, wobei in einem wässrigen Milieu das Peptid keine alpha-Helix aufweist.

**9.** Das Peptid nach einem der Ansprüche 1 bis 8, wobei das Peptid am C-Terminus als Carbonsäureamid ausgebildet ist.

**10.** Das Peptid nach einem der Ansprüche 1 bis 9 mit $3 \leq x, z \leq 7$ und $5 \leq y \leq 10$,

wobei A und C unabhängig voneinander ausgewählt sind aus der Gruppe bestehend aus Lys, Arg und Kombinationen davon und
wobei B ausgewählt ist aus der Gruppe bestehend aus Val, Ile, Phe und beliebigen Kombinationen von Val, Leu, Ile und Phe.

**11.** Das Peptid nach einem der Ansprüche 1 bis 10, wobei das Peptid mindestens 90% oder mindestens 95% Sequenzidentität mit einer Sequenz ausgewählt aus der Gruppe bestehend aus SEQ ID NOs: 1-13, 15, 16, 19-25 und 27-49 aufweist.

**12.** Das Peptid nach einem der Ansprüche 1 bis 11 zur Verwendung als Medikament.

**13.** Ein Konjugat umfassend das Peptid nach einem der Ansprüche 1 bis 11 und wenigstens eine weitere Komponente, an welche das Peptid kovalent und/oder adsorptiv gebunden vorliegt.

**14.** Eine antimikrobielle Zusammensetzung umfassend das Peptid nach einem der Ansprüche 1 bis 11.

**Claims**

**1.** A peptide with an amino-terminal region A, a carboxy-terminal region C and, in between, a region B of the general formula

$$A_x B_y C_z,$$

with $x, z \geq 1$, wherein $x \geq 3$ and/or $z \geq 3$, $y \geq 3$ and $x + y + z \leq 50$,
wherein A and C, in each case independently of one another, have at least 65%, 75% or 80% basic amino acids,
wherein B has at least 80% hydrophobic and/or nonpolar amino acids selected from the group consisting of Val, Ile, Phe and any combinations of Val, Leu, Ile and Phe, and comprises a consecutive sequence of at least three, four or five hydrophobic amino acids.

**2.** The peptide according to claim 1 with $y \geq 4$ or $y \geq 5$.

**3.** The peptide according to any one of claims 1 or 2, wherein the amino-terminal amino acid in A and/or the carboxy-terminal amino acid in C is a basic amino acid.

**4.** The peptide according to any one of claims 1 to 3, wherein the basic amino acids, independently of one another, are selected from the group consisting of Lys, Arg and His or from Lys and Arg.

**5.** The peptide according to any one of claims 1 to 4, wherein A and/or C comprise exclusively basic amino acids and/or wherein B comprises exclusively hydrophobic and/or nonpolar amino acids.

**6.** The peptide according to any one of claims 1 to 5 with $y \leq 20$, $y \leq 15$ or $y \leq 10$ and/or with $x + y + z \leq 30$ or $x + y + z \leq 20$.

**7.** The peptide according to any one of claims 1 to 6,
wherein the peptide is formed at least partially as beta strand in an aqueous medium, preferably wherein at least 50%, 65%, 75% or 80% of the amino acids of the peptide are comprised in a single beta strand.

**8.** The peptide according to any one of claims 1 to 7, wherein the peptide has no alpha-helix in an aqueous medium.

**9.** The peptide according to any one of claims 1 to 8, wherein the peptide on the C-terminus is formed as carboxamide.

**10.** The peptide according to any one of claims 1 to 9, with $3 \leq x, z \leq 7$ and $5 \leq y \leq 10$,

wherein A and C, independently of one another, are selected from the group consisting of Lys, Arg and combinations thereof and

wherein B is selected from the group consisting of Val, Ile, Phe and any combinations of Val, Leu, Ile, Phe.

**11.** The peptide according to any one of claims 1 to 10, wherein the peptide has at least 90% or at least 95% sequence identity with a sequence selected from the group consisting of SEQ ID NOs: 1-13, 15, 16, 19-25 and 27-49.

**12.** The peptide according to any one of claims 1 to 11 for use as medicament.

**13.** A conjugate comprising the peptide according to any one of claims 1 to 11 and at least one further component to which the peptide is covalently and/or adsorptively bonded.

**14.** An antimicrobial composition comprising the peptide according to any one of claims 1 to 11.

**Revendications**

**1.** Peptide comprenant une région amino-terminale A, une région carboxy-terminale C et une région intercalaire B de formule générale

$$A_xB_yC_z,$$

dans laquelle $x$, $z \geq 1$, sachant que $x \geq 3$ et/ou $z \geq 3$, $y \geq 3$ et $x + y + z \leq 50$,
A et C comportant chacun indépendamment l'un de l'autre au moins 65 %, 75 % ou 80 % d'acides aminés basiques,
B comportant au moins 80 % d'acides aminés hydrophobes et/ou non polaires issus de l'ensemble constitué de Val, Ile, Phe et de toutes combinaisons de Val, Leu, Ile et Phe et comprenant une séquence directe d'au moins trois, quatre ou cinq acides aminés hydrophobes.

**2.** Peptide selon la revendication 1, dans lequel $y \geq 4$ ou $y \geq 5$.

**3.** Peptide selon l'une des revendications 1 ou 2, dans lequel l'acide aminé amino-terminal en A et/ou l'acide aminé carboxy-terminal en C sont des acides aminés basiques.

**4.** Peptide selon l'une des revendications 1 à 3, dans lequel les acides aminés basiques sont choisis indépendamment les uns des autres dans l'ensemble constitué de Lys, Arg, et His ou de Lys et Arg.

**5.** Peptide selon l'une des revendications 1 à 4, dans lequel A et/ou C comprend exclusivement des acides aminés basiques et/ou dans lequel B comprend exclusivement des acides aminés hydrophobes et/ou non polaires.

**6.** Peptide selon l'une des revendications 1 à 5, dans lequel $y \leq 20$, $y \leq 15$ ou $y \leq 10$, et/ou dans lequel $x + y + z \leq 30$ ou $x + y + z \leq 20$.

**7.** Peptide selon l'une des revendications 1 à 6, dans lequel le peptide est conçu au moins partiellement comme un brin bêta, dans un milieu aqueux, de préférence au moins 50 %, 65 %, 75 % ou 80 % des acides aminés du peptide sont compris dans un seul brin bêta.

**8.** Peptide selon l'une des revendications 1 à 7, dans lequel le peptide ne présente pas d'hélice alpha dans un milieu aqueux.

**9.** Peptide selon l'une des revendications 1 à 8, dans lequel le peptide à l'extrémité C-terminale est conçu comme un amide d'acide carboxylique.

**10.** Peptide selon l'une des revendications 1 à 9, dans lequel $3 \leq x$, $z \leq 7$ et $5 \leq y \leq 10$,
A et C étant choisis indépendamment l'un de l'autre dans l'ensemble constitué de Lys, Arg, et de combinaisons de ceux-ci, et
B étant choisi dans l'ensemble constitué de Val, Ile, Phe et de toutes combinaisons de Val, Leu, Ile et Phe.

**11.** Peptide selon l'une des revendications 1 à 10, dans lequel le peptide présente au moins 90 % ou au moins 95 % d'identité séquentielle avec une séquence choisie dans l'ensemble constitué de SEQ ID NO : 1-13, 15, 16, 19-25 et 27-49.

**12.** Peptide selon l'une des revendications 1 à 11 destiné à être utilisé comme médicament.

**13.** Conjugué comprenant le peptide selon l'une des revendications 1 à 11 et au moins un autre composant auquel le peptide est relié par covalence et/ou adsorption.

**14.** Composition antimicrobienne comprenant le peptide selon l'une des revendications 1 à 11.

**Figur 1**

Fluoreszenz ex. 488 nm / em. 525 nm

**Figur 2**

**Figur 3**

**Figur 4**

**Figur 5**

**Figur 6**

**Figur 7**

**Figur 8**

**Figur 9**

N1    N2    M1    N3    N4

**Figur 10**

C1    C2    M1    C3    C4

**Figur 11**

H1    M1    H2    H3    H8

**Figur 12**

A1    A2    A3    A4    A5

**Figur 13**

**Figur 14**

**Figur 15**

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Nicht-Patentliteratur

- **AHMAD, A. ; AZMI, S. ; SRIVASTAVA, R. M. ; SRIVASTAVA, S. ; PANDEY, B. K. ; SAXENA, R. ; BAJPAI, V. K. ; GHOSH, J. K.** Design of nontoxic analogues of cathelicidin-derived bovine antimicrobial peptide BMAP-27: the role of leucine as well as phenylalanine zipper sequences in determining its toxicity. *Biochemistry,* 2009, vol. 48, 10905-17
- **BELL, G. ; GOUYON, P. H.** Arming the enemy: the evolution of resistance to selfproteins. *Microbiology,* 2003, vol. 149, 1367-75
- **BROGDEN, K. A. ; ACKERMANN, M. ; MCCRAY, P. B., JR. ; TACK, B. F.** Antimicrobial peptides in animals and their role in host defences. *Int J Antimicrob Agents,* 2003, vol. 22, 465-78
- **BROGDEN, K. A.** Antimicrobial peptides: pore formers or metabolic inhibitors in bacteria?. *Nat Rev Microbiol,* 2005, vol. 3, 238-50
- **BROTZ, H. ; BIERBAUM, G. ; LEOPOLD, K. ; REYNOLDS, P. E. ; SAHL, H. G.** The lantibiotic mersacidin inhibits peptidoglycan synthesis by targeting lipid II. *Antimicrob Agents Chemother,* 1998, vol. 42, 154-60
- Performance standards for antimicrobial susceptibility testing; sixteenth informational supplement. CLSI document M100-S16CLSI. Clinical and Laboratory Standards Institute, 2006
- **CONLON, J. M. ; SONNEVEND, A. ; PATEL, M. ; CAMASAMUDRAM, V. ; NOWOTNY, N. ; ZILAHI, E. ; IWAMURO, S. ; NIELSEN, P. F. ; PÁL, T.** A melittin-related peptide from the skin of the Japanese frog, Rana tagoi, with antimicrobial and cytolytic properties. *Biochemical and Biophysical Research Communications,* 2003, vol. 306, 496-500
- **DATHE, M. ; WIEPRECHT, T. ; NIKOLENKO, H. ; HANDEL, L. ; MALOY, W. L. ; MACDONALD, D. L. ; BEYERMANN, M. ; BIENERT, M.** Hydrophobicity, hydrophobic moment and angle subtended by charged residues modulate antibacterial and haemolytic activity of amphipathic helical peptides. *FEBS Lett,* 1997, vol. 403, 208-12
- **DIAMOND, G.** Natures antibiotics: the potential of antimicrobial peptides as new drugs. *Biologist (London),* 2001, vol. 48, 209-12
- Determination of minimum inhibitory concentrations (MICs) of antibacterial agents by broth dilution. **CLINICAL, M. ; INFECTIOUS, D.** Clinical Microbiology and Infection. European Committee for Antimicrobial Susceptibility Testing of the European Society, 2003, vol. 9, ix-xv
- **GAUTIER, R. ; DOUGUET, D. ; ANTONNY, B. ; DRIN, G.** HELIQUEST: a web server to screen sequences with specific alpha-helical properties. *Bioinformatics,* 2008, vol. 24, 2101-2
- **GENNARO, R. ; ZANETTI, M.** Structural features and biological activities of the cathelicidin-derived antimicrobial peptides. *Biopolymers,* 2000, vol. 55, 31-49
- **GINSBURG, I. ; KOREN, E.** Are cationic antimicrobial peptides also 'double-edged swords'?. *Expert Rev Anti Infect Ther,* 2008, vol. 6, 453-62
- **GIULIANI, A. ; PIRRI, G. ; NICOLETTO, S.** Antimicrobial peptides: an overview of a promising class of therapeutics. *Central European Journal of Biology,* 2007, vol. 2, 1-33
- **GORDON YJ FAU ; ROMANOWSKI, E. G. ; ROMANOWSKI EG FAU ; MCDERMOTT, A. M. ; MCDERMOTT, A. M.** A review of antimicrobial peptides and their therapeutic potential as anti-infective drugs. *Curr Eye Res,* 2005, vol. 30, 505-515
- **HILPERT, K. ; MCLEOD, B. ; YU, J. ; ELLIOTT, M. R. ; RAUTENBACH, M. ; RUDEN, S. ; BURCK, J. ; MUHLE-GOLL, C. ; ULRICH, A. S. ; KELLER, S.** Short cationic antimicrobial peptides interact with ATP. *Antimicrob Agents Chemother,* 2010, vol. 54, 4480-3
- **JIN, Y. ; HAMMER, J. ; PATE, M. ; ZHANG, Y. ; ZHU, F. ; ZMUDA, E. ; BLAZYK, J.** Antimicrobial activities and structures of two linear cationic peptide families with various amphipathic beta-sheet and alpha-helical potentials. *Antimicrob Agents Chemother,* 2005, vol. 49, 4957-64
- **KAUR, H. ; GARG, A. ; RAGHAVA, G. P.** PEPstr: a de novo method for tertiary structure prediction of small bioactive peptides. *Protein Pept Lett,* 2007, vol. 14, 626-31
- **LEHRER, R. I. ; GANZ, T.** Defensins of vertebrate animals. *Curr Opin Immunol,* 2002, vol. 14, 96-102
- Bioanalytik. Spektrum Akademischer Verlag, 2006
- **MARR, A. K. ; GOODERHAM, W. J. ; HANCOCK, R. E. W.** Antibacterial peptides for therapeutic use: obstacles and realistic outlook. *Current Opinion in Pharmacology,* 2006, vol. 6, 468-472
- **MAUPETIT, J. ; DERREUMAUX, P. ; TUFFERY, P.** PEP-FOLD: an online resource for de novo peptide structure prediction. *Nucleic Acids Res,* 2009, vol. 37, W498-503

- **MAUPETIT, J. ; DERREUMAUX, P. ; TUFFERY, P.** A fast method for large-scale de novo peptide and miniprotein structure prediction. *J Comput Chem,* 2010, vol. 31, 726-38
- **ONG, Z. Y. ; GAO, S. J. ; YANG, Y. Y.** Short Synthetic β-Sheet Forming Peptide Amphiphiles as Broad Spectrum Antimicrobials with Antibiofilm and Endotoxin Neutralizing Capabilities. *Advanced Functional Materials,* 2013, vol. 23, 3682-3692
- **OTVOS, L., JR., O, I. ; ROGERS, M. E. ; CONSOL-VO, P. J. ; CONDIE, B. A. ; LOVAS, S. ; BULET, P. ; BLASZCZYK-THURIN, M.** Interaction between heat shock proteins and antimicrobial peptides. *Biochemistry,* 2000, vol. 39, 14150-9
- **OYSTON, P. C. ; FOX, M. A. ; RICHARDS, S. J. ; CLARK, G. C.** Novel peptide therapeutics for treatment of infections. *J Med Microbiol,* 2009, vol. 58, 977-87
- **PUG, U. ; OEDENKOVEN, M. ; PAPO, N. ; OREN, Z. ; SHAI, Y. ; SAHL, H.-G.** In vitro activity and mode of action of diastereomeric antimicrobial peptides against bacterial clinical isolates. *Journal of Antimicrobial Chemotherapy,* 2004, vol. 53, 230-239
- **PETTERSEN, E. F. ; GODDARD, T. D. ; HUANG, C. C. ; COUCH, G. S. ; GREENBLATT, D. M. ; MENG, E. C. ; FERRIN, T. E.** UCSF Chimera-A visualization system for exploratory research and analysis. *Journal of Computational Chemistry,* 2004, vol. 25, 1605-1612
- **RAUSCH, J. M. ; MARKS, J. R. ; WIMLEY, W. C.** Rational combinatorial design of pore-forming beta-sheet peptides. *Proc Natl Acad Sci U S A,* 2005, vol. 102, 10511-5
- **RAUSCH, J. M. ; MARKS, J. R. ; RATHINAKUMAR, R. ; WIMLEY, W. C.** Beta-sheet pore-forming peptides selected from a rational combinatorial library: mechanism of pore formation in lipid vesicles and activity in biological membranes. *Biochemistry,* 2007, vol. 46, 12124-39
- **RICHTER, G.** Praktische Biochemie. Georg Thieme Verlag, 2003
- **SHAI, Y.** Mechanism of the binding, insertion and destabilization of phospholipid bilayer membranes by alpha-helical antimicrobial and cell nonselective membrane-lytic peptides. *Biochim Biophys Acta,* 1999, vol. 1462, 55-70
- **STRYER, L.** Biochemie. Spektrum Akademischer Verlag, 1999
- **SUBBALAKSHMI, C. ; SITARAM, N.** Mechanism of antimicrobial action of indolicidin. *FEMS Microbiol Lett,* 1998, vol. 160, 91-6
- **THEVENET, P. ; SHEN, Y. ; MAUPETIT, J. ; GUY-ON, F. ; DERREUMAUX, P. ; TUFFERY, P.** PEP-FOLD: an updated de novo structure prediction server for both linear and disulfide bonded cyclic peptides. *Nucleic Acids Res,* 2012, vol. 40, W288-93
- **THOMPSON, J.D. ; HIGGINS, D.G. ; GIBSON, T.J.** CLUSTAL W: improving the sensitivity of progressive multiple sequence alignment through sequence weighting, position-specific gap penalties and weight matrix choice. *Nucleic Acid Research,* 1994, vol. 22, 4673-4680
- **TOSSI, A. ; SANDRI, L. ; GIANGASPERO, A.** Amphipathic, alpha-helical antimicrobial peptides. *Biopolymers,* 2000, vol. 55, 4-30
- **WIEGAND, I. ; HILPERT, K. ; HANCOCK, R. E.** Agar and broth dilution methods to determine the minimal inhibitory concentration (MIC) of antimicrobial substances. *Nat Protoc,* 2008, vol. 3, 163-75
- **WIRADHARMA, N. ; SNG, M. Y. S. ; KHAN, M. ; ONG, Z. Y. ; YANG, Y. Y.** Rationally Designed alpha-Helical Broad-Spectrum Antimicrobial Peptides with Idealized Facial Amphiphilicity. *Macromolecular Rapid Communications,* 2013, vol. 34, 74-80
- **ZAIOU, M.** Multifunctional antimicrobial peptides: therapeutic targets in several human diseases. *J Mol Med (Berl),* 2007, vol. 85, 317-29